(19) **Europäisches Patentamt** / **European Patent Office** / **Office européen des brevets**

(11) **EP 1 769 741 B1**

(12) # EUROPEAN PATENT SPECIFICATION

(45) Date of publication and mention
of the grant of the patent:
**29.05.2013 Bulletin 2013/22**

(21) Application number: **05745882.0**

(22) Date of filing: **31.05.2005**

(51) Int Cl.:
**A61B 5/04** (2006.01)

(86) International application number:
**PCT/JP2005/009928**

(87) International publication number:
**WO 2005/117695 (15.12.2005 Gazette 2005/50)**

(54) **CARDIAC MAGNETIC FIELD DIAGNOSTIC APPARATUS AND DAMAGED CARDIAC MUSCLE THREE-DIMENSIONAL LOCALIZATION EVALUATING METHOD**

KARDIALES MAGNETFELD-DIAGNOSEGERÄT UND DREIDIMENSIONALES VERFAHREN ZUR UNTERSUCHUNG DER LOKALISATION EINES GESCHÄDIGTEN HERZMUSKELS

APPAREIL DE DIAGNOSTIC DE CHAMP MAGNÉTIQUE CARDIAQUE ET MÉTHODE D'ÉVALUATION DE LOCALISATION TRIDIMENSIONNELLE DE MUSCLE CARDIAQUE ENDOMMAGÉ

(84) Designated Contracting States:
**DE IT**

(30) Priority: **01.06.2004 JP 2004162980**
**10.09.2004 JP 2004263703**

(43) Date of publication of application:
**04.04.2007 Bulletin 2007/14**

(73) Proprietor: **National University Corporation Iwate University**
**Morioka-shi**
**Iwate 020-8550 (JP)**

(72) Inventors:
• **NAKAI, Kenji**
**0200113 (JP)**
• **KAWAZOE, Kohei**
**Morioka-shi,**
**Iwate 0200011 (JP)**
• **KOBAYASHI, Koichiro**
**0200114 (JP)**
• **ITO, Manabu**
**0200015 (JP)**
• **NAKAMURA, Yoshihiko**
**0200025 (JP)**
• **SHIMIZU, Takayuki**
**Morioka-shi,**
**Iwate 0200103 (JP)**
• **YOSHIZAWA, Masahito**
**0200116 (JP)**

(74) Representative: **Hofer, Dorothea et al**
**Prüfer & Partner GbR**
**Patentanwälte**
**Sonckestrasse 12**
**81479 München (DE)**

(56) References cited:
**EP-A1- 1 302 160    JP-A- 11 128 191**
**JP-A- 2001 309 899    US-A1- 2003 120 163**
**US-A1- 2004 082 870    US-B1- 6 187 032**

• **KATILA T ET AL: "On the accuracy of source localisation in cardiac measurements (magnetocardiography)" PHYSICS IN MEDICINE AND BIOLOGY, TAYLOR AND FRANCIS LTD. LONDON, GB, vol. 32, no. 1, 1 January 1987 (1987-01-01), pages 125-131, XP020023155 ISSN: 0031-9155**
• **FENICI R R ET AL: "CLINICAL MAGNETOCARDIOGRAPHY. 10 YEARS EXPERIENCE AT THE CATHOLIC UNIVERSITY" INTERNATIONAL JOURNAL OF CARDIAC IMAGING, DORDRECHT, NL, vol. 7, no. 3-4, 1991, pages 151-167, XP009082445 ISSN: 0167-9899**
• **KILLMANN R ET AL: "LOCALISATION OF MYOCARDIAL ISCHAEMIA FROM THE MAGNETOCARDIOGRAM USING CURRENT DENSITY RECONSTRUCTION METHOD: COMPUTER SIMULATION STUDY" MEDICAL AND BIOLOGICAL ENGINEERING AND COMPUTING, SPRINGER, HEILDELBERG, DE, vol. 33, no. 5, 1 September 1995 (1995-09-01), pages 643-651, XP000530851 ISSN: 0140-0118**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**Technical Field**

The present invention relates to a cardiac magnetic field diagnostic apparatus and an evaluating method of three-dimensional localization of myocardial injury, and more particularly, to a cardiac magnetic field diagnostic apparatus that calculates a three-dimensional distribution of current densities of the heart from a cardiac magnetic field of a subject so as to configure a cardiac magnetic-field integral cubic diagram (cardiac contour cubic diagram), enables cardiac spatial recognition or configuration of an excitation propagating locus, and reconfigures the three-dimensional localization of a myocardial injury in the same space of the subject, and an evaluating method of three-dimensional localization of myocardial injury.

**Background Art**

Diagnosis of a myocardial injury is important for diagnosis of diseases of coronary arteries such as cardiac infarction, because the lesion of the coronary arteries can be estimated by determining the localization of the myocardial injury.

As conventional diagnosing methods of the myocardial injury, the following methods are used. For example, nuclear medicine methods using a single photon of Thallium-201 or Tc-99m tetrofosmin or radioactive isotope (RI) $^{18}$F-FDG or NH3 are golden standards.

Further, contrast echocardiography with a contrast medium and evaluation of the myocardial injury using an MRI method with a gadolinium (Gd) contrast medium have recently been proposed.

All the methods use the contrast medium applied to the radioactive isotope, ultrasonic waves, or magnetic resonance method, and are invasive for the living body.

In addition, the above-mentioned conventional diagnosing methods cannot display the absolute position of the myocardial injury on the three-dimensional space.

Recently, it is well known that a re-entry circuit serving as an abnormal excitation propagating circuit is formed in the myocardium, thereby causing various arrhythmias (WPW (Wolff-Parkinson White), atrial flutter, atrial fibrillation, and the like) of various cardiac diseases.

Recent development of medical operations such as catheter cauterization enables radical treatment with respect to the arrhythmias.

For treatment of the arrhythmias, preferably, the formed portion of the re-entry circuit in the myocardium, serving as pathogeny, is identified with a noninvasive method. However, the invasive method such as Electro-anatomical mapping method with a catheter is conventionally used.

An SQUID fluxmeter using a Superconducting Quantum Interference Device (hereinafter, referred to as SQUID) for detecting nano magnetic flux of terrestrial magnetism with high sensitivity is applied to various fields. In particular, in biometry that greatly needs noninvasive measurement, the human body undergoes contactless magnetic measurement with the SQUID fluxmeter.

Especially, the recent advance of a technology for producing a thin film element results in development of a DC-SQUID Accordingly, magnetocardiography serving as a distribution of cardiac magnetic-fields is measured with the SQUID fluxmeter. Since this measurement of the cardiac magnetic-field is not affected by the constitution of the lung or torso-shaped organ, it is characterized that the cardiac magnetic-field generated by a cardiac electrical phenomenon can be three-dimensionally analyzed.

Further, methods for obtaining a three-dimensional distribution of current densities in the myocardium from a two-dimensional distribution of magnetic fields of the heart with the SQUID fluxmeter are proposed (refer to Japanese Patent Laying-Open No. 2002-28143 (Patent Document 1), Japanese Patent Laying-Open No. 2002-28144 (Patent Document 2), Japanese Patent Laying-Open No. 2002-28145 (Patent Document 3), Kenji NAKAI et al., "Specification of Infarcted and Ischemic Myocardium by Synthetic Aperture Magnetometry on Magnetocardiography", Japanese Journal of Electro cardiology (2003), vol. 23, No. 1, pages 35-44 (Non-Patent Document 1), Kenji NAKAI et al., "Visualization of Origin of Source by Spatial Filter Method on Magnetocardiography", Japanese Journal of Electrocardiology (2004), vol. 24, No. 1, pages 59-66 (Non-Patent Document 2), Masato YOSHIZAWA et al., "Current Density Imaging of MCG signal by Modified SAM", Collected Papers of Conference of Japan Biomagnetism and Bioelectromagnetics Society, 2002; 15; 109 (Non-Patent Document 3), M. Yoshizawa et al. "Current density imaging of simulated MCG signal by Modified Synthetic Aperture Magnetometry", BIOMAG 2002, August 2002, (Germany) (Non-Patent Document 4), and Kenji NAKAI et al., "Clinical Application and Utility of Magnetocardiography", Heart, vol. 36, No. 7, pages 549-555, Heart Editing Committee, published on July 15, 2004 (Non-Patent Document 5)).

These method are proposed for estimating a signal source of abnormal excitation propagation and estimating the viable myocardium on the basis of the measured cardiac magnetic-field with an estimation method of the distribution of current densities using, e.g., Synthetic Aperture Magnetometry (hereinafter, referred to as SAM) Further, these methods are proposed for estimating the distribution of current densities from the distribution of cardiac magnetic-fields with a new spatial filter having an excellent spatial resolution obtained by least square of Tikhonov normalization.

Patent Document 1: Japanese Patent Laying-Open No. 2002-28143
Patent Document 2: Japanese Patent Laying-Open No. 2002-28144
Patent Document 3: Japanese Patent Laying-Open No. 2002-28145
Non-Patent Document 1: Kenji NAKAI et al., "Specification of Infarcted and Ischemic Myocardium by Synthetic Aperture Magnetometry on Magnetocardiography", Japanese Journal of Electrocardiology (2003), vol. 23, No. 1, pages 35-44
Non-Patent Document 2: Kenji NAKAI et al., "Visualization of Origin of Source by Spatial Filter Method on Magnetocardiography", Japanese Journal of Electrocardiology (2004), vol. 24, No. 1, pages 59-66
Non-Patent Document 3: Masato YOSHIZAWA et al., "Current Density Imaging of MCG signal by Modified SAM", Collected Papers of Conference of Japan Biomagnetism and Bioelectromagnetics Society, 2002;15; 109
Non-Patent Document 4: M. Yoshizawa et al. "Current density imaging of simulated MCG signal by Modified Synthetic Aperture Magnetometry", BIOMAG 2002, August 2002, (Germany)
Non-Patent Document 5: Kenji NAKAI et al., "Clinical Application and Utility of Magnetocardiography", Heart, vol. 36, No. 7, pages 549-555, Heart editing committee, published on July 15, 2004

## Disclosure of the Invention

### Problems to be Solved by the Invention

For determination of the myocardial injury, in particular, the infarcted myocardium, the following information obtained with the above-mentioned magnetocardiography is advantageous.

First, QRS waves in a magnetocardiography reflect a cardiac electromotive force, and analysis of the QRS waves in the magnetocardiography is important for the determination of the myocardial injury.

Further, T waves in the magnetocardiography reflect a repolarization process of the myocardium, and analysis of a T-wave vector (direction of the repolarization process) in the magnetocardiography is important for the determination of the myocardial injury.

Furthermore, the dispersion of RT time, i.e., RT-dispersion in the magnetocardiography reflects a variation of a repolarization time of the myocardium (the time difference between max and min), and analysis of the RT-dispersion in the magnetocardiography is important for the determination of the myocardial injury.

Conventionally, with the magnetocardiography, the QRS waves, T-wave vector, and RT-dispersion are analyzed. Signals in the magnetocardiography are limited to two-dimensional information because of an actuarial reason of an inverse problem solution and only relatively spatial information on the two-dimension about the myocardial injury is obtained.

Further, as mentioned above, there are proposed the methods for obtaining the three-dimensional distribution of the current densities in the myocardium from the distribution of cardiac magnetic-fields with the spatial filter (refer to Patent Documents 1 to 3 and Non-Patent Documents 1 to 5). Since the three-dimensional space of the heart of the same case cannot be recognized, the absolute localization of the myocardial injury on the three-dimensional space cannot be determined.

Furthermore, in the above-mentioned cardiac magnetic-field measurement with the conventional SQUID fluxmeter, data indicating the three-dimensional distribution of the current densities in the myocardium from the distribution of the measured cardiac magnetic-fields is calculated, and a positional relationship of an abnormal excitation propagating circuit in the myocardium is identified (refer to, e.g., Patent Documents 1 to 3).

However, the three-dimensional space of the heart cannot be anatomically recognized with respect to data on the obtained three-dimensional distribution of the current densities. Further, the generated portion of the abnormal excitation propagating circuit in the myocardium, serving as the cause of the arrhythmia, on the three-dimension cannot be anatomically recognized with accuracy.

In particular, with respect to a subject, an anatomical image additionally-obtained with an MRI method or CT method tries to be reconstructed to the data on the three-dimensional distribution of the current densities, obtained by the above-mentioned distribution of the cardiac magnetic-fields. However, two pieces of data obtained at different times with different methods cannot precisely be matched each other without the spatial difference and the heart cannot be spatially recognized.

EP-1 302 160 A discloses a cardiac magnetic-field diagnostic apparatus for performing three-dimensional localization of a myocardial injury, comprising cardiac magnetic-field distribution measuring means that generates data on a two-dimensional distribution of a cardiac magnetic-field corresponding to a plurality of coordinates on the chest of a subject with contactless magnetic measurement of the plurality of co-ordinates; current-density data generating means that generates data on a three-dimensional distribution of current densities of the myocardium of the subject on the basis of the generated data on the two-dimensional distribution of the cardiac magnetic-field; and myocardial injury data generating means that generates data indicating the three-dimensional localization of a myocardial injury of the heart on the basis of the data on the three-dimensional distribution of the current densities.

It is one object of the present invention to provide a cardiac magnetic-field diagnostic apparatus that can configure a cardiac contour cubic diagram from the distribution of current densities in the myocardium, obtained with the measurement of the cardiac magnetic-field and can evaluate the three-dimensional localization of a myocardial injury in the configured three-dimensional space.

Further, it is another object of the present invention to provide an evaluating method of three-dimensional localization of a myocardial injury that can configure a cardiac contour cubic diagram from a distribution of current densities in the myocardium, obtained with the measurement of the cardiac magnetic-field and can evaluate the three-dimensional localization of the myocardial injury in the configured three-dimensional space.

Furthermore, it is another object of the present invention to provide a cardiac magnetic-field diagnostic apparatus that can draw a cardiac contour from a distribution of current densities in the myocardium, obtained with the measurement of the cardiac magnetic-field and can anatomically recognize the space of the heart

In addition, it is another object of the present invention to provide a cardiac magnetic-field diagnostic apparatus that can draw a cardiac contour from a distribution of current densities in the myocardium, obtained with measurement of the cardiac magnetic-field and can configure the excitation propagating locus of the heart.

## Means for Solving the Problems

According to one aspect of the present invention, a cardiac magnetic-field diagnostic apparatus for performing three-dimensional localization of a myocardial injury, comprises: cardiac magnetic-field distribution measuring means that generates data on a two-dimensional distribution of a cardiac magnetic-field corresponding to a plurality of coordinates on the chest of a subject with contactless magnetic measurement of the plurality of coordinates; current-density data generating means that generates data on a three-dimensional distribution of current densities of the myocardium of the subject on the basis of the generated data on the two-dimensional distribution of the cardiac magnetic-field; cardiac cubic diagram structuring means that structures a cardiac magnetic-field integral cubic diagram indicating a cardiac contour on the basis of the data on the three-dimensional distribution of the current densities; myocardial injury data generating means that generates data indicating the three-dimensional localization of a myocardial injury of the heart on the basis of the data on the three-dimensional distribution of the current densities; and image restructuring means that restructures the three-dimensional localization of the myocardial injury on the same space as that of the structured cardiac magnetic-field integral cubic diagram.

Preferably, the myocardial injury data generating means comprises: difference calculating means that obtains the QRS difference between average data of pre-obtained data on a three-dimensional distribution of the current densities of QRS waves of a plurality of healthy individuals and data on a three-dimensional distribution of the current densities of QRS waves of the subject; and drawing data generating means that generates data for drawing the three-dimensional localization of the myocardial injury on the basis of the obtained QRS difference.

Preferably, the difference calculating means of the QRS difference comprises: integrating means that obtains an integral value for a period of the QRS waves of the data on the three-dimensional distribution of the current densities at the three-dimensional coordinates of the chest of the subject; data storing means that obtains and stores an average of the integral values for the period of the QRS waves of the plurality of healthy individuals, obtained by the integrating means; and arithmetic operation means that obtains, as the QRS difference, the difference between an average of the integral values of the data on the three-dimensional distribution of the current densities at the three-dimensional coordinates of the chest of the healthy individual and an integral value of the data of the three-dimensional distribution of the current densities of the subject.

Preferably, the drawing data generating means comprises: means that colors, with predetermined colors, points each corresponding to one of the three-dimensional coordinates on the basis of the value of the QRS difference on the coordinates; means that linearly interpolates an interval between points corresponding to the three-dimensional coordinates; and means that performs perspective projection of the linearly-interpolated three-dimensional coordinate space.

Preferably, the drawing data generating means sets the degree of transparency of the color at each of the coordinates in accordance with the size of the QRS difference.

Preferably, the myocardial injury data generating means comprises: vector angle calculating means that obtains an angle of a current vector from data on a three-dimensional distribution of the current densities of T waves of the subject; and drawing data generating means that generates data for drawing the three-dimensional localization of the myocardial injury on the basis of the obtained angle of the current vector of the T waves.

Preferably, the vector angle calculating means comprises: first integrating means that obtains an integral value for a period of the T waves of an X component of the data on the three-dimensional distribution of the current densities at the three-dimensional coordinates of the chest of the subject; second integrating means that obtains an integral value for a period of the T waves of a Y component of the data on the three-dimensional distribution of the current densities at the three-dimensional coordinates of the chest of the subject; and arithmetic operation means that obtains the angle of the current vector from a ratio of the integral values of the X component and the Y component of the data on the three-dimensional distribution of the current densities at the three-dimensional coordinates on the chest of the subject.

Preferably, the drawing data generating means comprises: means that colors, with predetermined colors, points each corresponding to one of the three-dimensional coordinates on the basis of the angle of the current vector at the coordinates; means that linearly interpolates an interval between the points corresponding to the three-dimensional coordinates; and means that performs perspective projection of the linearly-interpolated three-dimensional coordinate space.

Preferably, the drawing data generating means sets the degree of transparency of the color of each of the points at the coordinates in accordance with the size of the angle of the current vector.

Preferably, the myocardial injury data generating means comprises: time distribution calculating means that obtains an RT-dispersion, as a distribution of RT time, from data on a three-dimensional distribution of the current densities of QRS-T waves of the subject; and drawing data generating means that generates data for drawing the three-dimensional localization of the myocardial injury on the basis of the obtained RT-dispersion.

Preferably, the time distribution calculating means comprises: means that obtains, as the RT-dispersion, an absolute value of the difference between a maximum value and a minimum value of the RT time from the data on the three-dimensional distribution of the current densities at the three-dimensional coordinates on the chest of the subject.

Preferably, the drawing data generating means comprises: means that colors, with predetermined colors, point each corresponding to one of the three-dimensional coordinates on the basis of the RT-dispersion at the coordinates; means that linearly interpolates an interval between the points corresponding to the three-dimensional coordinates; and means that performs perspective projection of the linearly interpolated three-dimensional space.

Preferably, the drawing data generating means sets the degree of transparency of the color of each of the coordinates in accordance with the size of the RT-dispersion.

Preferably, the cardiac cubic diagram structuring means comprises: integrating means that obtains an integral value for a predetermined period of data on the three-dimensional distribution of the current densities at the three-dimensional coordinates of the chest of the subject, or of data on three-dimensional energy density, obtained by squaring the data on the three-dimensional distribution of the current densities; maximum-value determining means that obtains a maximum value of the integral values at the coordinates; cube setting means that segments the three-dimensional coordinate of the chest into a plurality of sets of cubes; threshold setting means that sets a threshold on the basis of the maximum value of the integral values; and high/low determining means that determines whether the integral value at the coordinates corresponding to a vertex of the cube is higher or lower than the set threshold; image generating means that generates, as the cardiac magnetic-field integral cubic diagram, an image displaying the high/low determination result of the integral value in the set of a plurality of cubes.

Preferably, the image generating means comprises: means that calculates the number of vertexes having the integral value at the corresponding coordinates higher than the threshold among eight vertexes forming the cube for each of the plurality of cubes; means that draws a polygon for connecting a vertex higher than the threshold in a predetermined form in accordance with the number of vertexes having the integral value higher than the threshold; and means that aligns the plurality of cubes in the three-dimensional space of the chest and performs perspective projection of the drawn polygon, and the polygon set of the cubes obtained by the perspective projection forms the cardiac magnetic-field integral cubic diagram.

According to another aspect of the present invention, an evaluating method of three-dimensional localization of a myocardial injury, comprises: a step of generating data on a two-dimensional distribution of a cardiac magnetic-field corresponding to a plurality of coordinates of the chest of a subject with contactless magnetic measurement; a step of generating data on a three-dimensional distribution of current densities of the myocardium of the subject on the basis of the generated data on the two-dimensional distribution of the cardiac magnetic-field; a step of structuring a cardiac magnetic-field integral cubic diagram indicating a cardiac contour on the basis of the data on the three-dimensional distribution of the current densities; a step of generating data indicating three-dimensional localization of the myocardial injury of the heart on the basis of the data on the three-dimensional distribution of the current densities; and a step of restructuring the three-dimensional localization of the myocardial injury on the same space as that of the structured cardiac magnetic-field integral cubic diagram.

Preferably, the step of generating the data indicating the three-dimensional localization of the myocardial injury comprises: a step of obtaining the QRS difference between average data of pre-obtained data on the three-dimensional distribution of the current densities of QRS waves of a plurality of healthy individuals and data on the three-dimensional distribution of the current densities of the QRS waves of the subject; and a step of generating data for drawing the three-dimensional localization of the myocardial injury on the basis of the obtained QRS difference.

Preferably, the step of obtaining the QRS difference comprises: a step of obtaining an integral value for a period of the QRS waves of the data on the three-dimensional distribution of the current densities at the three-dimensional coordinates of the chest of the subject; a step of obtaining and storing an average value of the integral values for the QRS waves of the plurality of healthy individuals obtained in the step of obtaining the integral value; and a step of obtaining, as the QRS difference, the difference between the average of the integral values of the data on the three-dimensional distribution of the current densities of the chest of the healthy individual on the three-dimensional coordinates and the integral value of the data on the three-dimensional distribution of the current densities of the subject.

Preferably, the step of generating the drawing data comprises: a step of coloring, with predetermined colors, point each corresponding to one of the three-dimensional coordinates on the basis of a value of the QRS difference on the coordinates; a step of linearly interpolating an interval between the points corresponding to the three-dimensional coordinates; and a step of performing perspective projection of the linearly-interpolated three-dimensional coordinate space.

Preferably, the step of generating the drawing data comprises: a step of setting the degree of transparency of the color at each of the coordinates in accordance with the size of the QRS difference.

Preferably, the step of generating the data indicating the three-dimensional localization of the myocardial injury comprises: a step of obtaining an angle of a current vector from the data on the three-dimensional distribution of the current densities of T waves of the subject; and a step of generating data for drawing the three-dimensional localization of the myocardial injury on the basis of the obtained angle of the current vector of the T waves.

Preferably, the step of obtaining the vector angle comprises: a step of obtaining an integral value for a period of the T waves of an X component of the data on the three-dimensional distribution of the current densities at the three-dimensional coordinates of the chest of the subject; a step of obtaining an integral value for a period for the T waves of a Y component of the data on the three-dimensional distribution of the current densities at the three-dimensional coordinates of the chest of the subject; and a step of obtaining the angle of the current vector from a ratio of the integral values of the X component and Y component of the data on the three-dimensional distribution of the current densities at the three-dimensional coordinates of the chest.

Preferably, the step of generating the drawing data comprises: a step of coloring, with predetermined colors, points each corresponding to the three-dimensional coordinates on the basis of the angle of the current vector at the coordinates; a step of linearly interpolating an interval between the points corresponding to the three-dimensional coordinates; and a step of performing perspective projection of the linearly interpolated three-dimensional coordinate space.

Preferably, the step of generating the drawing data comprises: a step of setting the degree of transparency of the color on each of the coordinates in accordance with the size of the angle of the current vector.

Preferably, the step of generating the data indicating the three-dimensional localization of the myocardial injury comprises: a step of obtaining RT-dispersion, as a distribution ofRT time from data on three-dimensional distribution of the current densities of QRS-T waves of the subject; and a step of generating data for drawing the three-dimensional localization of the myocardial injury on the basis of the obtained RT-dispersion.

Preferably, the step of obtaining the RT-dispersion comprises: a step of obtaining, as the RT-dispersion, an absolute value of the difference between a maximum value and a minimum value of the RT time from the data on the three-dimensional distribution of the current densities at the three-dimensional coordinates of the chest of the subject.

Preferably, the step of generating the drawing data comprises: a step of coloring, with predetermined colors, points each corresponding to one of the three-dimensional coordinates on the basis of the RT-dispersion on the coordinate; a step of linearly interpolating an interval between of the points corresponding to the three-dimensional coordinates; and a step of performing perspective projection of the linearly-interpolated three-dimensional space.

Preferably, the step of generating the drawing data comprises: a step of setting the degree of transparency of the color on each of the coordinates in accordance with the size of the RT-dispersion.

Preferably, the step of structuring the cardiac magnetic-field integral cubic diagram comprises: a step of obtaining an integral value for a predetermined period of the data on the three-dimensional distribution of the current densities at the three-dimensional coordinates of the chest of the subject, or of data on three-dimensional energy density, obtained by squaring the data on the three-dimensional distribution of the current densities; a step of obtaining a maximum value of the integral values on the coordinate; a step of segmenting the three-dimensional coordinate of the chest into a plurality of sets of cubes; a step of setting a threshold on the basis of the maximum value of the integral values; a step of determining whether the integral value at the coordinate corresponding to a vertex of the cube is higher or lower than the set threshold; and a step of generating, as the cardiac magnetic-field integral cubic diagram, an image displaying the high/low determination result of the integral value in the set of the plurality of cubes.

Preferably, the step of generating the image comprises: a step of calculating the number of vertexes having the integral value on the corresponding coordinate higher than the threshold among eight vertexes forming the cube for each of the plurality of cubes; a step of drawing a polygon for connecting a vertex having the integral value higher than the threshold in a predetermined form in accordance with the number of vertexes having the integral value higher than the threshold; and a step of aligning the plurality of cubes in the three-dimensional space of the chest and performs perspective projection of the drawn polygon, and the polygon set of the cubes obtained by the perspective projection forms the cardiac magnetic-field integral cubic diagram.

According to another aspect of the present invention, a cardiac magnetic-field diagnostic apparatus comprises: cardiac magnetic-field distribution measuring means that generates data on a two-dimensional distribution of a cardiac magnetic-field corresponding to a plurality of coordinates with contactless magnetic measurement of the chest of a subject; first arithmetic-operation means that generates data on a three-dimensional distribution of the current densities of the myocardium of the subject on the basis of the generated data on the two-dimensional distribution of the cardiac magnetic-field; second arithmetic-operation means that structures a cardiac magnetic-field integral cubic diagram indicating a cardiac contour on the basis of the data on the three-dimensional distribution of the current densities; magnetic signal recognizing means that generates a predetermined magnetic field applied externally at a predetermined position on the chest of the subject, and recognizes the predetermined position on the chest; and spatial position identifying means that identifies the recognized predetermined position on the same space as that of the structured cardiac magnetic-field integral cubic diagram.

Preferably, the second arithmetic-operation means comprises: integrating means that obtains an integral value for a predetermined period of the data on the three-dimensional distribution of the current densities at the three-dimensional coordinates of the chest of the subject or of data on three-dimensional energy density, obtained by squaring the data on the three-dimensional distribution of the current densities; maximum-value determining means that obtains a maximum value of the integral values on the coordinates; cube setting means that segments the three-dimensional coordinates of the chest into a plurality of sets of cubes; threshold setting means that sets a threshold on the basis of the maximum value of the integral value; and high/low determining means that determines whether the integral value on the coordinate corresponding to a vertex of the cubic is higher or lower than the set threshold; and image generating means that generates, as the cardiac magnetic-field integral cubic diagram, an image displaying the high/low determination result of the integral value in the set of the plurality of cubes.

Preferably, the image generating means comprises: means that calculates the number of vertexes having the integral value at the corresponding coordinates, higher than the threshold, among eight vertexes forming the cube for each of the plurality of cubes; means that draws a polygon for connecting a vertex having the integral value higher than the threshold in a predetermined form in accordance with the number of vertexes having the integral value higher than the threshold; and means that aligns the plurality of cubes in the three-dimensional space of the chest and performs perspective projection of the drawn polygon, and the polygon set of the cubes obtained by the perspective projection forms the cardiac magnetic-field integral cubic diagram.

Preferably, the predetermined period corresponds to a time of the atrium portion of P waves, upon obtaining a magnetic-field integral cubic diagram indicating an atrium contour of the heart.

Preferably, the predetermined period corresponds to a time of the ventricle portion of QRS waves, upon obtaining a magnetic-field integral cubic diagram indicating a ventricle contour of the heart.

Preferably, the cardiac magnetic-field diagnostic apparatus further comprises: means that supplies an anatomical image of the chest of the subject, having the predetermined position that is specified; and means that combines the anatomical image with the cardiac magnetic-field integral cubic diagram, having the predetermined position that is identified.

According to another aspect of the present invention, a cardiac magnetic-field diagnostic apparatus comprises: cardiac magnetic-field distribution measuring means that generates data on a two-dimensional distribution of a cardiac magnetic-field corresponding to a plurality of coordinates on the chest of a subject with contactless magnetic measurement on the plurality of coordinates; first arithmetic-operation means that generates data on a three-dimensional distribution of current densities of the myocardium of the subject on the basis of the generated data on the two-dimensional distribution of the cardiac magnetic-field; second arithmetic-operation means that structures a cardiac magnetic-field integral cubic diagram indicating a cardiac contour on the basis of the data on the three-dimensional distribution of the current densities; third arithmetic-operation means that structures a three-dimensional excitation propagating locus of an impulse conducting system in the myocardium of the subject on the basis of the data on the three-dimensional distribution of the current densities; and data combining means that combines the structured cardiac magnetic-field integral cubic diagram with the structured three-dimensional excitation propagating locus.

Preferably, the second arithmetic-operation means comprises: integrating means that obtains an integral value for a predetermined period of the data on the three-dimensional distribution of the current densities at the three-dimensional coordinates of the chest of the subject, or of data on three-dimensional energy density, obtained by squaring the data on the three-dimensional distribution of the current densities; maximum-value determining means that obtains a maximum value of the integral value at the coordinates; cube setting means that segments the three-dimensional coordinates of the chest into a plurality of sets of cubes; threshold setting means that sets a threshold on the basis of the maximum value of the integral value; and high/low determining means that determines whether the integral value of the coordinates corresponding to a vertex of the cube is higher or lower than the set threshold; and image generating means that generates, as the cardiac magnetic-field integral cubic diagram, an image displaying the high/low determination result of the integral value in the set of the plurality of cubes.

Preferably, the image generating means comprises: means that calculates the number of vertexes having the integral value at the corresponding coordinates, higher than the threshold, among eight vertexes forming the cube for each of the plurality of cubes; means that draws a polygon for connecting a vertex having the integral value higher than the threshold in a predetermined form in accordance with the number of vertexes having the integral value higher than the threshold; and means that aligns the plurality of cubes in the three-dimensional space of the chest and performs perspective projection of the drawn polygon, and the polygon set of the cubes obtained by the perspective projection forms the cardiac magnetic-field integral cubic diagram.

Preferably, the third arithmetic-operation means comprises: means that obtains coordinates of the highest value of the data on the distribution of current densities at the three-dimensional coordinates of the chest of the subject, at a plurality of timings within the predetermined period; means that draws a line connecting the coordinates of the highest values at the plurality of timings; and means that repeats the operation for connecting the coordinates of the highest values while shifting the timings.

Preferably, the means for drawing the line connecting the highest values connects the coordinates with a B-spline curve.

Preferably, the predetermined period corresponds to a time of the atrium portion of P waves, upon obtaining a magnetic-field integral cubic diagram indicating an atrium contour of the heart.

Preferably, the predetermined period corresponds to a time of the ventricle portion of QRS waves, upon obtaining a magnetic-field integral cubic diagram indicating a ventricle contour of the heart.

Preferably, the cardiac magnetic-field diagnostic apparatus further comprises: means that supplies an anatomical image of the chest of the subject; and means that combines the anatomical image with the cardiac magnetic-field integral cubic diagram combined to the three-dimensional excitation propagating locus.

**Effects of the Invention**

As mentioned above, according to the present invention, from three-dimensional distribution of current densities of the chest of a subject, data relatively displaying a myocardial injury, such as QRS difference, T-wave vector, or RT-dispersion is displayed three-dimensionally and stereoscopically. Further, the data is reconstructed to a cardiac contour cubic diagram additionally-configured from three-dimensional distribution of current densities of the same subject, thereby enabling the absolute three-dimensional spatial display of the myocardial injury of the heart with noninvasiveness. The localization of the myocardial injury can be determined in diagnosis of a cardiac disease in a hospital or emergency room.

In particular, the present invention provides an advantageous method for diagnosing acute coronary syndromes (acute myocardial injury due to the decay of the atheroma of coronary arteries), which has been recently increased, and for evaluating coronary artery bypass grafting or coronary angioplasty with a catheter.

Further, according to the present invention, from the distribution of current densities in the myocardium calculated on the basis of noninvasive measurement of the cardiac magnetic-field, a cardiac magnetic-field integral cubic diagram is drawn as a cardiac contour, and the heart can be anatomically recognized on the space.

Furthermore, according to the present invention, from the distribution of current densities in the myocardium calculated on the basis of noninvasive the measurement of the cardiac magnetic-field, a cardiac magnetic-field integral cubic diagram is drawn as a cardiac contour, and an excitation propagating locus of the heart can be configured.

**Brief Description of the Drawings**

Fig. 1 is a diagram showing waveforms of magnetocardiography for illustrating the principle of the present invention.
Fig. 2 is a schematic block diagram showing the structure of a cardiac magnetic-field diagnostic apparatus according to first to third embodiments of the present invention.
Fig. 3 is a block diagram showing the specific structure of a magnetic-field distribution measurement device shown in Fig. 2.
Fig. 4 is a diagram showing an example of an alignment of a plurality of magnetic-field sensors on the front surface of the chest of a subject.
Fig. 5 is a diagram showing time-series data on magnetic fields obtained by the plurality of sensors shown in Fig. 4.
Fig. 6 is a diagram for schematically illustrating a method for calculating data on a current density from the time-series data on the magnetic field.
Fig. 7 is one flowchart for illustrating creating processing of a cardiac contour cubic diagram according to the first to fourth embodiments.
Fig. 8 is another flowchart for illustrating creating processing of the cardiac contour cubic diagram according to the first to fourth embodiments.

Fig. 9 is another flowchart for illustrating creating processing of the cardiac contour cubic diagram according to the first to fourth embodiments.

Fig. 10 is one schematic diagram conceptually showing a drawing method of a cardiac contour according to the present invention.

Fig. 11A is another schematic diagram conceptually showing the drawing method of the cardiac contour according to the present invention.

Fig. 11B is another schematic diagram conceptually showing the drawing method of the cardiac contour according to the present invention.

Fig. 11 C is another schematic diagram conceptually showing the drawing method of the cardiac contour according to the present invention.

Fig. 12A is another schematic diagram conceptually showing the drawing method of the cardiac contour according to the present invention.

Fig. 12B is another schematic diagram conceptually showing the drawing method of the cardiac contour according to the present invention.

Fig. 13A is another schematic diagram conceptually showing the drawing method of the cardiac contour according to the present invention.

Fig. 13B is another schematic diagram conceptually showing the drawing method of the cardiac contour according to the present invention.

Fig. 14 is another schematic diagram conceptually showing the drawing method of the cardiac contour according to the present invention.

Fig. 15 is another schematic diagram conceptually showing the drawing method of the cardiac contour according to the present invention.

Fig. 16 is a diagram showing a CT captured image showing the coil position on the body surface of a subject.

Fig. 17 is a waveform diagram of a signal from a coil measured with an SQUID fluxmeter.

Fig. 18 is a diagram showing the restructure of the coil position on the magnetocardiography using the SQUID fluxmeter.

Fig. 19 is a cardiac contour cubic diagram obtained according to the present invention.

Fig. 20 is a diagram showing an image obtained by reconstructing the cardiac contour cubic diagram shown in Fig. 19 with an MRI image.

Fig. 21 is a flowchart for illustrating display processing of the QRS difference according to the first embodiment of the present invention.

Fig. 22 is a flowchart for illustrating display processing of the QRS difference according to the first embodiment of the present invention.

Fig. 23A is one schematic diagram conceptually showing QRS-difference drawing processing shown in Fig. 22.

Fig. 23B is another schematic diagram conceptually showing the QRS-difference drawing processing shown in Fig. 22.

Fig. 24A is a diagram showing one actual example of three-dimensional display of the QRS difference of a healthy individual.

Fig. 24B is a diagram showing another example of the three-dimensional display of the QRS difference of the healthy individual.

Fig. 25A is a diagram showing one actual example of three-dimensional display of the QRS difference of a patient with myocardial injury.

Fig. 25B is a diagram showing another example of the three-dimensional display of the QRS difference in patient with myocardial injury.

Fig. 26A is a diagram showing one current vector measured according to the second embodiment of the present invention.

Fig. 26B is a diagram showing another current vector measured according to the second embodiment of the present invention.

Fig. 27 is a flowchart for illustrating one display processing of a T-wave vector according to the second embodiment of the present invention.

Fig. 28 is a flowchart for illustrating another display processing of the T-wave vector according to the second embodiment of the present invention.

Fig. 29 is a waveform diagram showing an additively-averaged waveform of magnetocardiography waveforms.

Fig. 30A is one schematic diagram conceptually showing T-wave vector drawing processing shown in Fig. 28.

Fig. 30B is another schematic diagram conceptually showing the T-wave vector drawing processing shown in Fig. 28.

Fig. 31 is a diagram showing a histogram of angular distribution of the T-wave vector. -

Fig. 32A is a diagram showing one actual example of three-dimensional display of the T-wave vector of the healthy individual.

Fig. 32B is a diagram showing another actual example of the three-dimensional display of the T-wave vector of the healthy individual.

Fig. 33A is a diagram showing one actual example of three-dimensional display of the T-wave vector in patient with myocardial injury.

Fig. 33B is a diagram showing another example of the three-dimensional display of the T-wave vector in patient with myocardial injury.

Fig. 34 is a diagram showing a circular graph of angular distribution of the T-wave vector.

Fig. 35 is a flowchart for illustrating one display processing of RT-dispersion according to the third embodiment of the present invention.

Fig. 36 is a flowchart for illustrating another display processing of the RT-dispersion according to the third embodiment of the present invention.

Fig. 37A is one schematic diagram conceptually showing RT-dispersion drawing processing shown in Fig. 36.

Fig. 37B is another schematic diagram conceptually showing the RT-dispersion drawing processing shown in Fig. 36.

Fig. 38A is a diagram showing one actual example of three-dimensional display of the RT-dispersion of the healthy individual.

Fig. 38B is a diagram showing another actual example of the three-dimensional display of the RT-dispersion of the healthy individual.

Fig. 39A is a diagram showing one actual example of three-dimensional display of the RT-dispersion in patient with myocardial injury.

Fig. 39B is a diagram showing another actual example of the three-dimensional display of the RT-dispersion in patient with myocardial injury.

Fig. 40 is a schematic block diagram showing the structure of a cardiac magnetic-field diagnostic apparatus according to the fourth embodiment of the present invention.

Fig. 41 is a flowchart for illustrating operation of the cardiac magnetic-field diagnostic apparatus according to the fourth embodiment of the present invention.

Fig. 42A is a diagram showing one example of the restructure of a cardiac contour cubic diagram to an excitation propagating locus, obtained according to the present invention.

Fig. 42B is a diagram showing another example of the restructure of the cardiac contour cubic diagram to the excitation propagating locus, obtained according to the present invention.

Fig. 43 is a diagram showing one example of the restructure of a spatially-recognized cardiac contour cubic diagram to the excitation propagating locus, obtained according to the present invention.

Fig. 44 is a diagram showing an image obtained by restructuring the cardiac contour cubic diagram and the excitation propagating locus shown in Fig. 43 to an MRI image.

## Description of the Reference Signs

1: magnetic-field distribution measurement device, 2: arithmetic operation unit, 3: anatomical image data generator, 4: display unit, 5: magnetic-field generator, 6: coil, 12: subject, 13: dewar, 14: arithmetic operation unit, 15: SQUID fluxmeter, 16: detecting coil, 17: coil, 18: SQUID element, 19: feedback coil, 20: Nb shield, 21: electrocardiograph, 22: storage device.

## Best Modes for Carrying Out the Invention

Hereinbelow, a specific description will be given of embodiments of the present invention with reference to the drawings. Incidentally, the same or similar portions in the drawings are designated by the same reference numerals and a description thereof is not repeated.

(First embodiment)

According to a first embodiment of the present invention, the QRS difference of magnetocardiography can be three-dimensionally displayed, thereby enabling the three-dimensional localization of a myocardial injury.

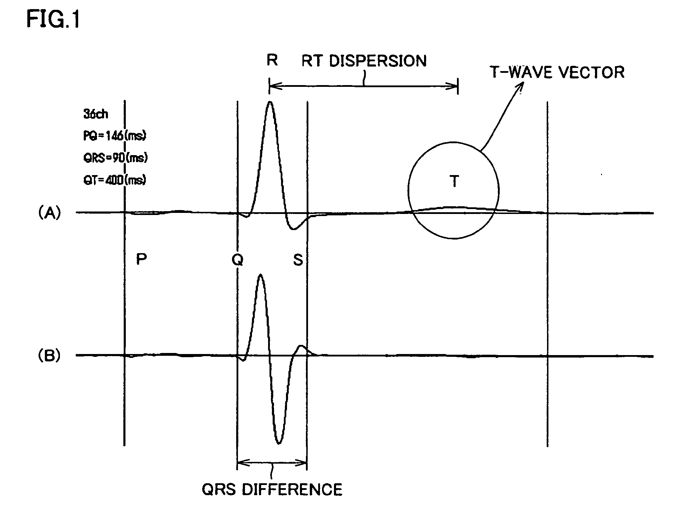

Fig. 1 is a waveform diagram showing actual waveforms of the magnetocardiography. Referring to Fig. 1, a description will be given of the principle of the first embodiment of the present invention.

In the actual waveforms of the magnetocardiography shown in Fig. 1, a waveform (A) corresponds to an actual waveform diagram of each channel of a cardiac magnetic-field measured with an SQUID fluxmeter, and a waveform (B) corresponds to a waveform diagram showing the QRS difference, which will be described later.

As mentioned above, the QRS waves reflect a cardiac electromotive force, and it is identified that the cardiac electromotive force is reduced at the portion where the myocardial injury such as cardiac infarction is caused. Therefore, three-dimensional distribution of current densities is obtained from a portion corresponding to the QRS waves of a magnetocardiography signal and the cardiac electromotive force is estimated, thereby enabling the determination of the localization of the myocardial injury.

According to the first embodiment of the present invention, average data of the three-dimensional distribution of the current densities is obtained in advance with a spatial filter from the portion corresponding to the QRS waves of magnetocardiography signals of a plurality of (e.g., 30) healthy individuals (hereinafter, referred to as a target group) without an obvious cardiac disease, and the resultant data is stored. Further, three-dimensional current density distribution is obtained with the spatial filter from the portion corresponding to the QRS waves of the magnetocardiography signal of a subject (patient) particularly having a cardiac disease such as myocardial infarction.

In addition, the difference between the average data on the three-dimensional distribution of the current densities of the target group in the QRS portion of a waveform and the data on the three-dimensional distribution of the current densities of the subject (hereinafter, referred to as the QRS difference) is obtained. This indicates spatial distribution of the myocardial injury such as myocardial infarction.

However, the acquisition of the difference between the data on the three-dimensional distribution of the current densities enables only the relative determination of the localization of the myocardial injury, and the absolute spatial localization of the heart on the three-dimension cannot be determined.

According to the first embodiment of the present invention, a cardiac contour can be drawn from the three-dimensional distribution of the current densities of the subject in the myocardium, obtained with the measurement of the cardiac magnetic-field, and the difference of the three-dimensional distribution of the current densities in the QRS wave portion between the target group and the subject is restructured in the space of the same subject as that of the drawn cardiac contour cubic diagram, thereby determining the absolute spatial localization of the myocardial injury on the three-dimension of the heart of the subject.

Hereinbelow, a description will be given of the specific structure for realizing the first embodiment of the present invention.

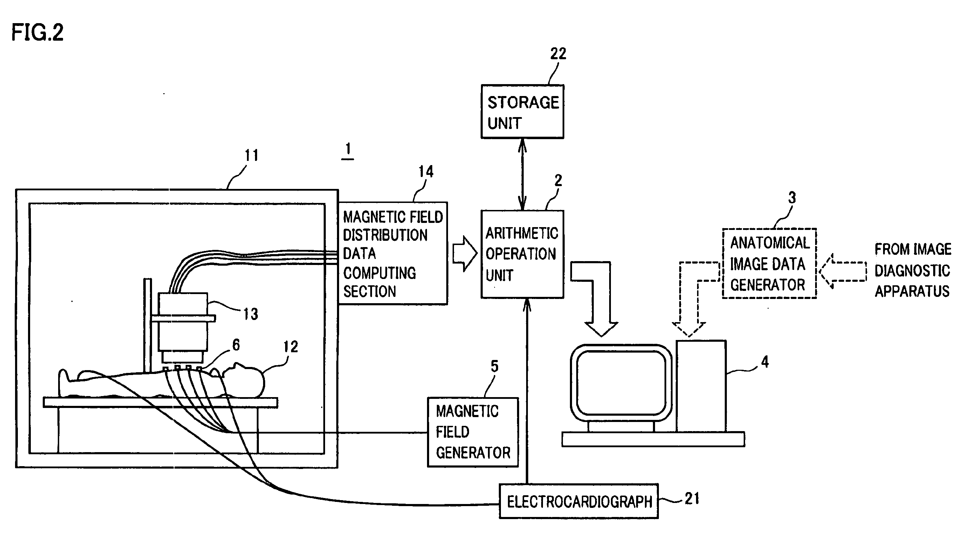

Fig. 2 is a block diagram showing the structure of a cardiac magnetic-field diagnostic apparatus according to the first embodiment of the present invention.

Referring to Fig. 2, a magnetic-field distribution measurement device 1 comprises a dewar 13 including an SQUID fluxmeter provided for contactless magnetic measurement on the chest of a subject 12 in a Magnetically Shielded Room (hereinafter, referred to as an MSR) 11, and a magnetic field distribution data computing section 14 of data on the distribution of magnetic fields, provided outside an MSR 11. Incidentally, the magnetic field distribution data computing section 14 of the data on the magnetic distribution may be provided in the MSR 11.

The dewar 13 includes an environment of a low-temperature system which is filled with liquid helium and superconductivity is thus generated. Further, the dewar 13 encloses an SQUID fluxmeter comprising a detecting coil formed of a superconductivity conductor.

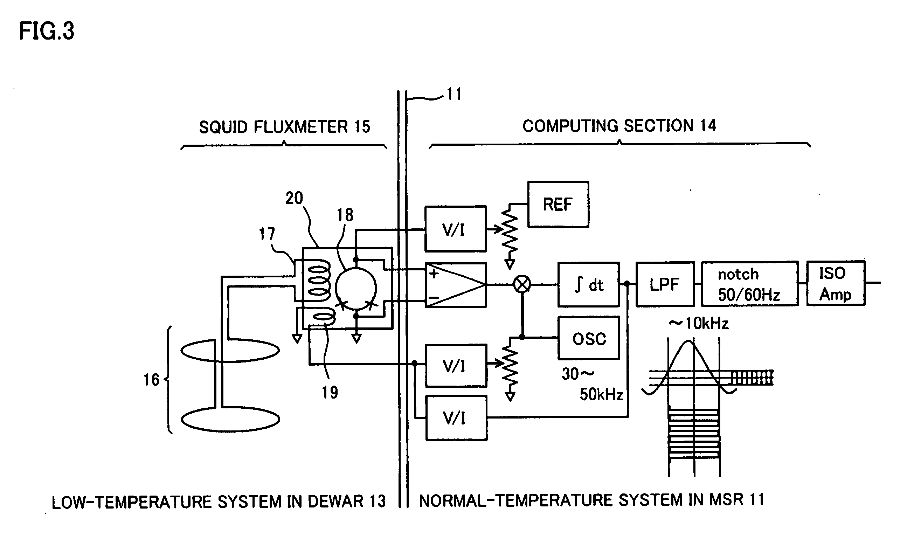

Fig. 3 is a block diagram specifically showing an SQUID fluxmeter 15 provided in the low-temperature system in the dewar 13 of the MSR 11 shown in Fig. 2 and the magnetic field distribution data computing section 14 provided at a room-temperature. Incidentally, as shown in Fig. 3, a modulation-system magnetic flux lock (FLL) system is used as the magnetic-field distribution data computing section 14, as will be described later. However, the magnetic-field distribution data computing section 14 may be a non-modulation system FLL.

The structure shown in Fig. 3 corresponds to one channel for measuring the data on the magnetic field at one point on the chest of the subject. As will be described later, the magnetic fields on a plurality of coordinates on the chest of the subject are simultaneously measured at multi-point according to the present invention. Therefore, the structure corresponding to one channel shown in Fig. 3 is provided corresponding to a plurality of channels necessary for measurement. In the following example, the magnetic fields are measured at 64 points of the coordinates of the chest of the subject, and the structure shown in Fig. 3 corresponding to 64 channels is provided.

Hereinbelow, a description is given of generating the data on the magnetic field corresponding to one channel with the SQUID fluxmeter with reference to Fig. 3.

First, the SQUID fluxmeter 15 includes a pickup coil 16 having superconductivity conductor that detects the magnetic field generated from the chest surface of the subject. When the pickup coil 16 applies the magnetic field, current flows. The current is transmitted to a coil 17, thereby generating the magnetic field in an Nb shield 20.

As a consequence, the magnetic field that linearly changes to the magnetic field is generated in an SQUID element 18. Proper bias current flows to the SQUID element 18, and voltages at both terminals of the SQUID element 18 are detected by an amplifier of the magnetic field distribution data computing section 14. The magnetic-field distribution data computing section 14 adjusts current flowing to a feedback coil 19 used for modulation of the magnetic field in the modulation FLL so as to prevent the change of the detected voltage, provided in the Nb shield 20.

That is, in the case of detecting the magnetic field of the living body with the SQUID, the generated magnetic field is not directly measured but the magnetic field detected with the pickup coil 16 is converted into an electrical signal with the magnetic-field distribution data computing section 14 and is further output by feedback operation (specifically, a constant magnetic field is generated in the SQUID element 18 by controlling the magnetic field generated in the feedback coil 19 with the adjustment of the current flowing to the feedback coil 19) with so-called zero null-balance method so as to keep a constant magnetic field of the SQUID element 18. In general, this feedback is a well-known as flux locked loop: hereinafter, referred to as FLL).

These SQUID fluxmeter 15 and magnetic field distribution data computing section 14 are well known technologies and a description thereof is thus omitted.

As mentioned above, the structure shown in Fig. 3 is necessary for measurement of the data on the magnetic field corresponding to one channel. With the structure, an electrical signal indicating time-series data on the magnetic field of the magnetic field measured at one point on the front surface of the chest of the subject is outputted.

According to the present invention, a large number of sensors (SQUID fluxmeters) are arranged on the front surface of the chest of the subject as mentioned above so as to measure the magnetic fields at the multi-point on the front surface of the chest. The magnetic field changes with time and, if the measurement place is different, the magnetic field differently changes depending on the place even during a period corresponding to, e.g., one heartbeat.

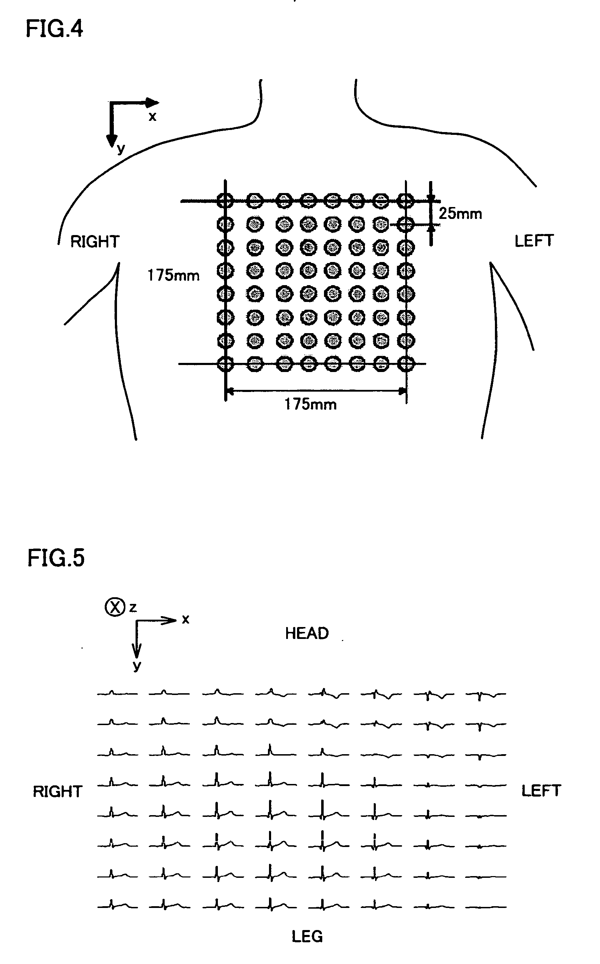

Fig. 4 is a diagram showing one example of the arrangement of a plurality of sensors (the SQUID fluxmeters, each corresponding to one channel) on the front surface of the chest of the subject. Fig. 5 is a diagram showing one group of time-series data on the magnetic field indicating the change of the magnetic field during one-heartbeat period at the positions of a plurality of sensors shown in Fig. 4, obtained by the sensors.

Data output from the magnetic-field distribution measurement device 1 shown in Fig. 2 indicates one group of the time-series data on the magnetic field corresponding to a plurality of measurement positions (coordinates) as shown in Fig. 5. One group of the time-series data on the magnetic field is picked-up by paying attention to a specific time point and it cannot expressed, as a graph (diagram), actual peaks and valleys indicating the distribution of strength of magnetic field at one time on the front surface of the chest as a measurement target. Accordingly, it is possible to obtain the data on the distribution of magnetic field expressed as a contour plot, like an atmospheric pressure on a weather map. In this viewpoint, the data output from the magnetic-field distribution measurement device 1 is considered as time-series data on the distribution of the magnetic fields on the front surface of the chest.

One group of time-series data on the magnetic field output from the magnetic-field distribution measurement device 1, that is, the time-series data on the distribution of the magnetic fields is sent to a arithmetic operation unit 2 shown in Fig. 2. The arithmetic operation unit 2 has a function for obtaining an electrical activity of the chest at one moment, e.g., the current density of the chest flowing at the moment on the basis of the data on the distribution of magnetic field at one time under software.

Further, the arithmetic operation unit 2 stores the resultant arithmetic data to a storage unit 22, as needed.

Hereinbelow, a description will be given of a method, with the arithmetic operation unit 2, for obtaining information on the electrical activity on the three-dimension of a portion (the heart in the present invention) in the human body, serving as a measurement target, e.g., the distribution of current densities flowing to the portion from the time-series data on the distribution of the magnetic fields generated by the magnetic-field distribution measurement device 1.

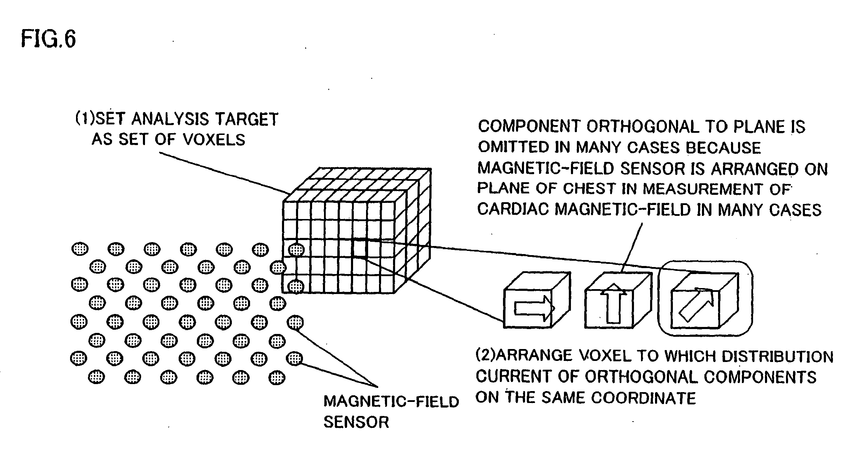

Fig. 6 is a schematic diagram illustrating the method for obtaining the current density. With the following method, if a current sensor (virtual sensor) is provided at one specific portion in the human body to be analyzed, the current that is to flow here is indirectly calculated. Accordingly, one coefficient is multiplied to the time-series data on the magnetic field obtained with all sensors (SQUID fluxmeters) provided on the front surface of the chest of the human body and the total thereof is obtained, thereby obtaining a current output of the virtual sensor. A main solution of this calculation is how the coefficient is obtained.

Hereinbelow, a detailed description will be given of the method for obtaining the current density with reference to Fig. 6. First, the total number, N magnetic-field sensors are arranged on the surface of the human body (front surface of the chest). The human body (the chest, particularly, heart), serving as an analysis target, is assumed as a collection set of voxels serving as small blocks. Herein, the total number of voxels is M.

Reference numeral Bj(t) denotes the time-series data on the magnetic field obtained with a sensor j, and reference numeral $\beta$ denotes a spatial filter coefficient of a voxel i corresponding to a sensor output Bj(t).

Herein, it is assumed that a virtual current sensor exists at a voxel i. In this case, reference numeral Si(t) denotes a virtual sensor output corresponding to the current density obtained with the virtual current sensor. In this case, Si(t) is defmed by the following expression.

[Expression 1]

$$Si(t) = \sum_{j=1}^{N} \beta_{ij} \cdot B_j(t)$$

Therefore, if the spatial filter coefficient $\beta_{ij}$ is determined, the current density at the voxel i is obtained. Further, the distribution of current densities on the three-dimension for the whole analysis target can be obtained.

As a method for setting the spatial filter coefficient $\beta_{ij}$ with a high sensitivity to the current distributed at the corresponding voxel i, various methods such as the above-mentioned SAM and MUSIC (Multiple Signal Classification) can be used. The SAM and MUSIC have been searched and developed in radar and sonar fields as being well-known.

The virtual sensor output, calculated in real-time, of the voxel obtained with the spatial filter coefficient according to the SAM or MUSIC has an advantage of extremely high real-time performance.

The SAM and MUSIC technologies are well known and algorithm for obtaining the spatial filter coefficient with these methods is extremely complicated. Therefore, a specific description thereof is omitted here. The SAM is described in detail in Robinson SE and Vrba J, "Functional Neuroimaging by Synthetic Aperture Magnetometry (SAM)" in "Recent Advances in Biomagnetism" (published by Tohoku University Press) in "Proceedings of the 11th International Conference on Biomagnetism" (1999), pages 302-305. The MUSIC is described in detail in Koh HARA and Shinya KURIKI, "Science of Cerebric Magetic field-SQIUD Measurement and Medical Applications" (on January 25, 1997, Ohmsha, Ltd.), pages 117-119.

As mentioned above, the arithmetic operation unit 2 generates the time-series data indicating the three-dimensional distribution of the current densities of the heart, serving as an analysis target, from the data on the distribution of magnetic fields generated with the magnetic-field distribution measurement device 1, and executes the calculation configuring a cardiac magnetic-field integral cubic diagram, which will be described later, under software.

With the configuring method of the cardiac magnetic-field integral cubic diagram according to the present invention, attention is paid to the fact that the current density basically exists only at the myocardium portion and the cardiac magnetic-field integral cubic diagram is configured, thereby assuming the configured cubic diagram as a cardiac contour.

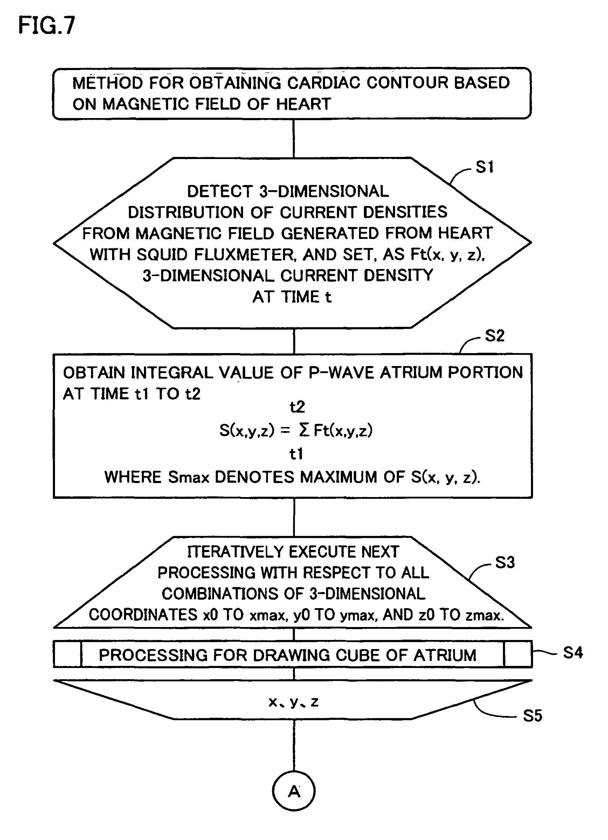

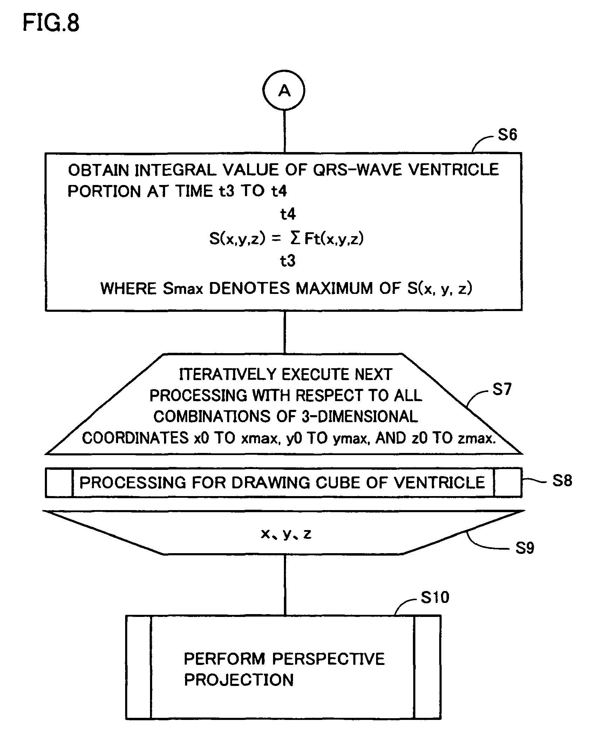

Figs. 7 and 8 are flowcharts of the configuring method of cardiac magnetic-field integral cubic diagram with the arithmetic operation unit 2 shown in Fig. 2 under software. In particular, Fig. 7 is a flowchart showing processing for drawing the cube of the atrium.

Referring to Fig. 7, in step S1, the three-dimensional current density is calculated from the distribution of the cardiac magnetic-fields detected with the SQUID fluxmeter shown in Fig. 2 according to the method using the spatial filter described above with reference to Fig. 6. Herein, reference numeral Ft(x, y, z) denotes the three-dimensional current density at three-dimensional coordinates x, y, and z of the chest of the subject, calculated at a time t. Incidentally, data between vertexes of the three-dimensional current densities undergoes linear interpolation.

Next, in step S2, S (x, y, z) serving as an integral value of the current density Ft (x, y, z) is obtained for a period from times t1 to t2 of the P-wave atrium portion measured with the electrocardiograph 21 shown in Fig. 2 with respect to coordinate points of all combinations of the three-dimensional coordinates x, y, and z. Further, Smax serving as a maximum value of S (x, y, z) is obtained.

Subsequently, steps S3, S4, and S5 indicate loop processing for drawing a magnetic-field integral cubic diagram of the cardiac atrium portion. The processing for drawing the cubic diagram of the atrium shown in step S4 is iteratively executed with respect to all combinations of three-dimensional coordinates x0 to xmax, y0 to ymax, and z0 to zmax shown in step S3 until closing the loop of x, y, and z in step S5.

Next, Fig. 8 is a flowchart showing processing for drawing the cube of the ventricle executed subsequently to the processing shown in Fig. 7 of the method for configuring the cardiac magnetic-field integral cubic diagram. Steps S6 to S9 in Fig. 8 are similar to the processing in steps S2 to S5 shown in Fig. 7, except for a point that the integration time in step S6 corresponds to times t3 to t4 of the QRS-wave ventricle portion measured with the electrocardiograph 21 and a description thereof is thus omitted.

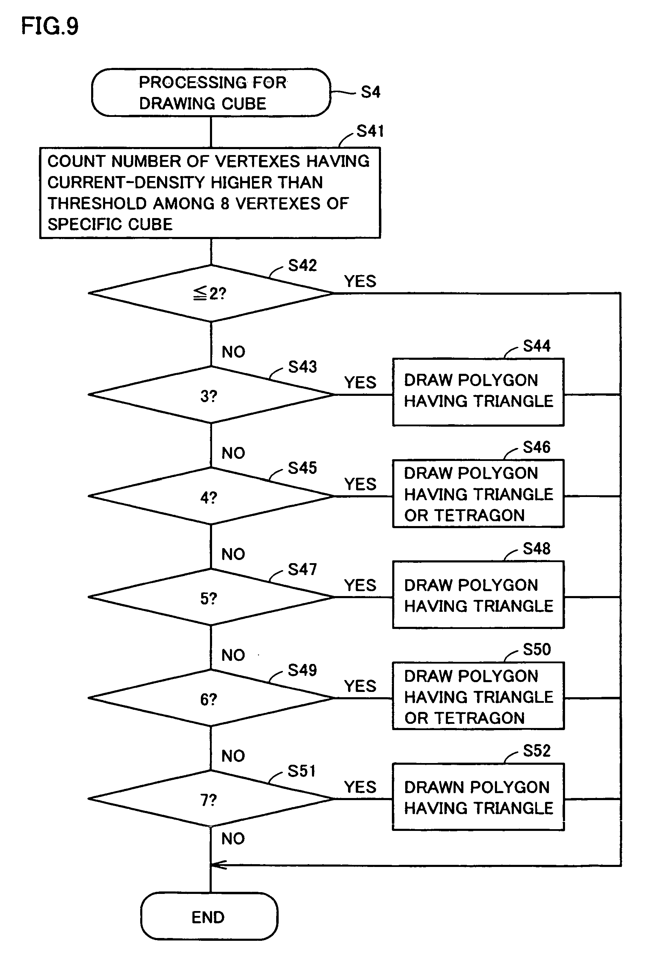

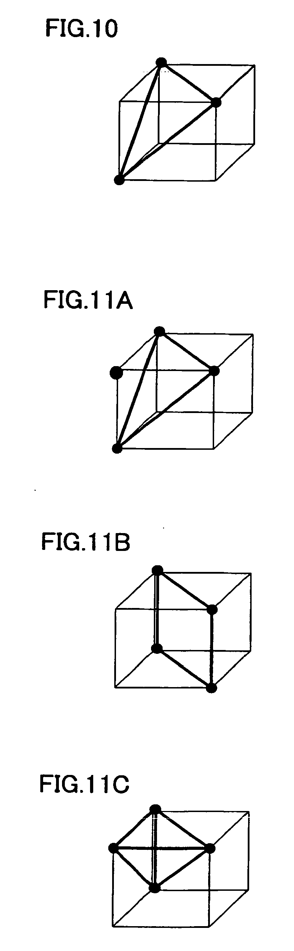

Fig. 9 is a flowchart showing common processing to the processing for drawing the cube of the atrium in step S4 in Fig. 7 and the processing for drawing the cubic of the ventricle in step S8 in Fig. 8. Further, Figs. 10 to Fig. 14 are schematic diagrams conceptually showing the processing for drawing the cube of the atrium or ventricle.

Hereinbelow, a description will be given of the processing for drawing the cube of the atrium in step S4 or the processing for drawing the cubic of the ventricle in step S8 with reference to Figs. 9 to Fig. 14.

First, the three-dimensional space of the chest of the subject is assumed as a plurality of cubes and, as one cube, a cubic having eight points of three-dimensional coordinates S (x, y, z), S (x+1, y, z), S (x, y+1, z), S (x, y, z+1), S (x+1, y+1, z), S (x+1, y, z+1), S (x, y+1, z+1), and S (x+1, y+1, z+1) as vertexes is assumed.

Further, a threshold is set on the basis of the maximum value Smax of the current density obtained in step S2 in Fig. 7. The threshold is set to accurately draw a cardiac contour diagram in consideration of a fact that strong and weak current densities in the myocardium exist.

The threshold is obtained by multiplying coefficients 0.0 to 1.0 to Smax, and 0.66666666 is used as an initial value of the coefficients. An operator of the device finely adjusts the coefficients to an optimum value while viewing the above-completed cubic diagram of the cardiac contour, as will be described later.

First, in step S41 in Fig. 9, among the 8 vertexes of the specific cube, the number of points having the integral value of the current density larger than the threshold based on the Smax is counted. Further, it is determined whether or not the number of vertexes is 2 or less (in step S42). If it is determined that the number of vertexes is 2 or less, any processing is not performed.

On the other hand, if it is determined that the number of vertexes is more than 2, it is subsequently determined whether or not the number of vertexes is 3 (in step S43). If it is determined that the number of vertexes is 3, in step S44, a polygon having triangles is drawn. That is, as shown in Fig. 10, a polygon connecting three vertexes having triangles is drawn.

On the other hand, if it is determined that the number of vertexes is not 3, it is subsequently determined whether or not the number of vertexes is 4 (in step S45). If it is determined that the number of vertexes is 4, in step S46, a polygon having triangles or tetragons is drawn.

That is, as shown in Fig. 11A, when one (large black circle) of four points is center and the remaining three points are adjacent to each other, a polygon connecting the three points having triangles is drawing.

Further, as shown in Fig. 11B, when 4 points are on the identical plane, a polygon connecting the 4 points having tetragons is drawn.

Furthermore, as shown in Fig. 11C, except for the foregoing, a polygon having four triangles is drawn.

On the other hand, if the number of vertexes is not 4, subsequently, it is determined whether or not the number of vertexes is 5 (in step S47). If it is determined that the number of vertexes is 5, in step S48, a polygon having triangles is drawn.

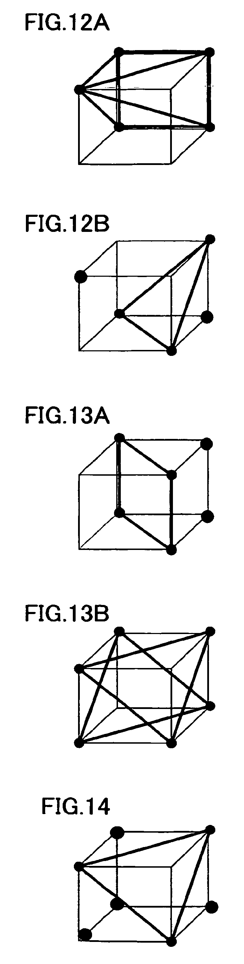

That is, as shown in Fig. 12A, a polygon consisting triangles connecting 5 points is drawn. Further, as shown in Fig. 12B, when 5 points are apart from each other, a polygon consisting triangles is drawn.

On the other hand, if it is determined that the number of vertexes is not 5, subsequently, it is determined whether or hot the number of vertexes is 6 (in step S49). If is determined that the number of vertexes is 6, in step S50, a polygon having triangles or tetragons is drawn.

That is, as shown in Fig. 13A, when two points having values not more than the threshold are on the identical side, a tetragonal polygon is drawn.

Further, as shown in Fig. 13B, when two points having values not more than the threshold are not on the identical side, a polygon having two triangles is drawn.

On the other hand, if the number of vertexes is not 6, subsequently it is determined whether or not the number of vertexes is 7 (in step S51). If it is determined that the number of vertexes is 7, in step S52, a polygon consisting triangles is drawn.

That is, as shown in Fig. 14, a polygon consisting triangles adjacent to one point having a value not more than the threshold is drawn.

On the other hand, if it is determined in step S51 that the number of vertexes is not 7, i.e., 8, any processing is not performed. Thus, the drawing of the polygons of one specific cube ends.

Further, in step S 10 in Fig. 8, perspective projection is performed by using all of the results of polygon drawing of the cube of the atrium, repeated in steps S3 to S5 in Fig. 7 and the results of polygon drawing of the cube of the ventricle, repeated in steps S7 to S9 in Fig. 8.

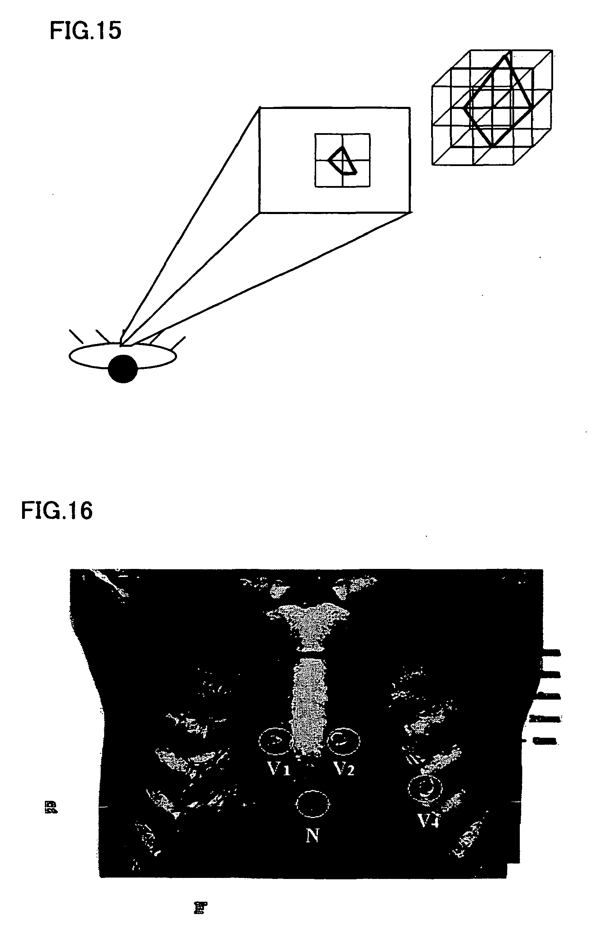

Fig. 15 is a diagram schematically showing the perspective projection in step S10. The set of polygons indicating the distribution of strong and weak current densities of the cube obtained as shown in Figs. 10 to Fig. 14 is subjected to the perspective projection, thereby obtaining image data of the magnetic-field integral cubic diagram of the myocardium. The image data is sent to one input of a display unit 4 shown in Fig. 2 and is drawn on the display. As mentioned above, the current density basically exists in the myocardium. Therefore, the above-obtained magnetic-field integral cubic diagram expresses a cubic diagram of the contour of the whole heart.

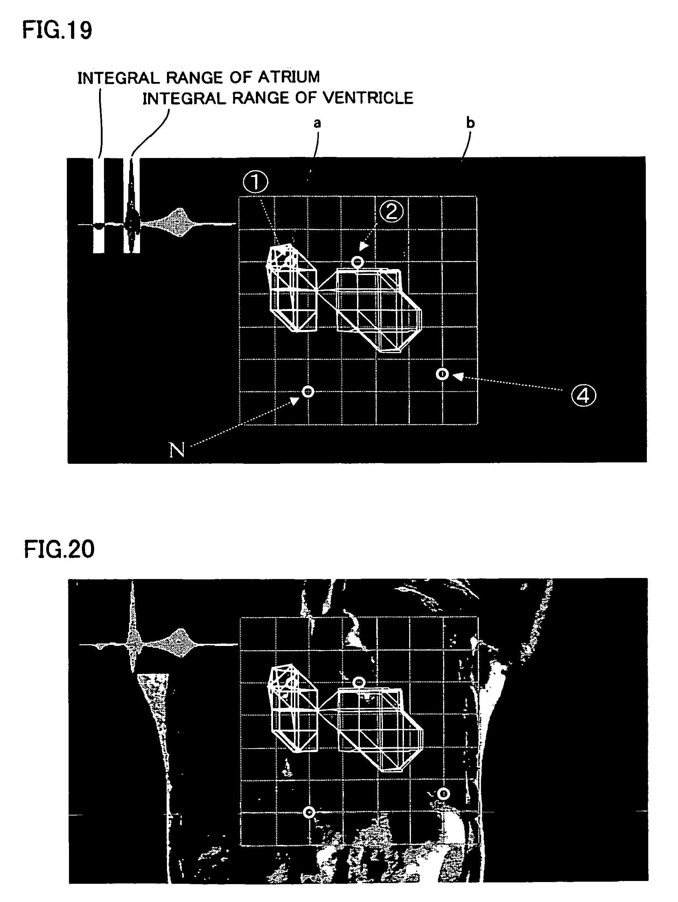

For example, the cardiac magnetic-field integral cubic diagram (solid-line frame shown by a line a on the left in the drawing) indicating the contour of the atrium portion and the cardiac magnetic-field integral cubic diagram (solid-line frame shown by a line b on the right in the drawing) indicating the contour of the ventricle portion at the coordinates of the 64 measurement points of the chest of the subject shown in Fig. 19 are drawn on the display of the display unit 4.

The final image is adjusted to the best state by finely adjusting the coefficients of the threshold while the operator views the image, as mentioned above.

Next, a description will be given of a method for spatially recognizing the cardiac contour expressed by the above-obtained cardiac magnetic-field integral cubic diagram.

That is, four magnetic-field coils 6 connected to a magnetic field generator 5 are provided to predetermined positions on the chest of the subject with reference to Fig. 2. In this example, the coils 6 are provided at four points including just right of the sternum at the level of the fourth intercostal space, just left of the sternum at the level of the fourth intercostal space, the midsternal line of the sternum at the level of the fifth intercostal space, and the ensiform cartilage.

Among the four points, the three points except for the ensiform cartilage correspond to an international standard lead point in a standard 12-lead electrocardiogram and can be a reference point in the standardization of the magnetocardiography leading method according to the present invention.

The four coils 6 generate the magnetic field in accordance with a predetermined signal supplied from the magnetic-field generator 5. The magnetic fields generated by the four coils 6 are detected by the SQUID fluxmeter included in the dewar 13.

Fig. 16 is a diagram showing the positions of the four coils 6 on the body surface of the chest of the subject on a CT captured image, and four circular marks in Fig. 16 indicate the coil positions. That is, reference numeral $V_1$ denotes the chest guided from just right of the sternum at the level of the fourth intercostal space, reference numeral $V_2$ denotes the chest guided from just left of the sternum at the level of the fourth intercostal space, reference numeral $V_4$ denotes the chest guided from the midsternal line of the sternum at the level of the fifth intercostal space, and reference numeral N denotes the ensiform cartilage.

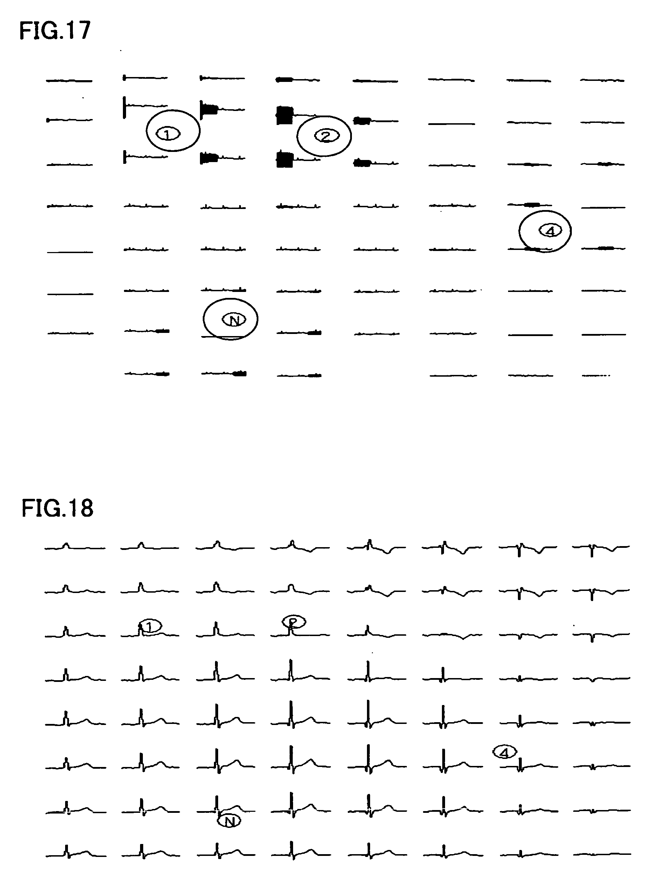

Next, Fig. 17 is a waveform diagram showing signals from the four coils on the body surface, measured with the SQUID fluxmeter having 64 channels. Referring to Fig. 17, reference numeral 1 denotes the chest guided from just right of the sternum at the level of the fourth intercostal space, reference numeral 2 denotes the chest guided from just left of the sternum at the level of the fourth intercostal space, reference numeral 4 denotes the chest guided from the midsternal line of the sternum at the level of the fifth intercostal space, and reference numeral N denotes the ensiform cartilage. The coil positions are identified by viewing the waveform diagram by the operator.

Fig. 18 is a diagram showing the state for restructuring the four coil positions on the magnetocardiography of the SQUID fluxmeter having 64 channels.

Further, the operator operates an input device (not shown) by visually recognizing the spatial positions of the coils from the magnetocardiography. As shown in Fig. 19, positions 1, 2, 4, and N are drawn with circular marks with respect to the four coils on the same space as that of the image indicating the cardiac contour cubic diagram on the display unit 4.

Herein, among the known four points (refer to Figs. 16 to 18) on the body surface of the subject, the points $V_1$, $V_2$, and N are on the identical plane. Although the point V4 varies depending on the subject, the point $V_4$ is in the depth of 1 to 2 cm. The cardiac contour cubic diagram displayed on the display unit 4 is switched to the display in the depth direction with the processing of the arithmetic operation unit 2, thereby three-dimensionally drawing even the coil positions having different depths in the contour cubic diagram.

As mentioned above, according to the present invention, the four points as the known coil positions are spatially associated with the cardiac magnetic-field integral cubic diagram, that is, the cardiac contour, drawn on the basis of the distribution of current densities obtained from the distribution of the cardiac magnetic-field detected with the SQUID fluxmeter from the cardiac magnetic-field, thereby enabling the recognition of the drawn cardiac spatial position.

In particular, according to the first embodiment of the present invention, with respect to the same subject, the cardiac contour cubic diagram measured with the same measurement method at the same time and the known coil positions are reconstructed on the same space. Therefore, as compared with the case of restructuring data conventionally-obtained by another method at another time, the heart can be spatially recognized with extreme accuracy without the spatial displacement.

If the heart can be spatially recognized with accuracy as mentioned, the combination to anatomical image data such as MRI or CT becomes easy as needed. Referring back to Fig. 2, if necessary, an anatomical image data generator 3 shown by a broken line receives slice image data on the chest of the same subject captured with another tomography diagnostic apparatus such as MRI or X-ray CT.

The anatomical image data generator 3 processes the received slice image data and performs three-dimensional perspective transformation from a predetermined viewpoint, thereby generating anatomical image data. The above-mentioned technology for generating the three-dimensional anatomical image from the slice image data is well known, as specifically disclosed in Japanese Patent Laying-Open No. 11-128224, PCT WO 98/15226 and the like. Therefore, a detailed description thereof is omitted.

As mentioned above, the anatomical image data generator 3 generates the data indicating the three-dimensional anatomical image of the chest near the heart of the same subject, and sends the resultant data to another input of the display unit 4.

The display unit 4 shown in Fig. 2 overlays an image indicating the cardiac contour formed based on the data on the cardiac magnetic-field integral cubic diagram from the arithmetic operation unit 2 on the three-dimensional anatomical image of the chest of the subject formed based on the data from the anatomical image data generator 3, and displays the resultant image.

Fig. 20 is a diagram showing the combination of the cardiac contour cubic diagram shown in Fig. 19 and the MRI image. By putting marks with a marker to the same four points as the four coils on the body surface of the same subject in MRI measurement, the combination to the cardiac contour cubic diagram can be accurately performed without spatial displacement.

According to the above-mentioned cardiac spatial recognizing method, the operator estimates the positions of four coils attached to the body surface are visually estimated from the level of the 64-channel magnetic-field waveforms obtained with the SQUID fluxmeter and the input means is operated, thereby drawing the magnetic-field coil positions on the same space as that of the cardiac contour cubic diagram. Instead of the viewing of the operator, obviously, the arithmetic operation unit 2 can determine the coil positions based on the output waveform of the 64-channel magnetic fluxmeter with signal processing of software and can draw the coil positions on the cardiac contour cubic diagram.

As mentioned above, according to the cardiac spatial recognizing method of the first embodiment in the present invention, the cardiac magnetic-field integral cubic diagram is drawn as a cubic diagram of the cardiac contour from the distribution of current densities in the myocardium calculated based on the noninvasive measurement of the cardiac magnetic field. As a consequence, the heart can be anatomically and spatially recognized with accuracy as mentioned.

In particular, with respect to the same subject, the cardiac contour cubic diagram measured at the same time according to the same measurement method and the known coil positions are restructured on the same space. Therefore, the heart can be spatially recognized with extreme accuracy without the spatial displacement therebetween.

As mentioned above, the arithmetic operation unit 2 generates the time-series data indicating the three-dimensional distribution of the current densities of the heart as the analysis target from the data on the distribution of magnetic fields generated by the magnetic-field distribution measurement device 1, and further generates the cardiac magnetic-field integral cubic diagram, i.e., the image data on the cardiac contour cubic diagram with the processing shown in Figs. 7 to Fig. 9.

According to the first embodiment of the present invention, thereafter, the arithmetic operation unit 2 performs processing for restructuring the QRS difference between the three-dimensional current densities in the above-obtained cardiac contour cubic diagram. That is, according to the first embodiment of the present invention, the QRS difference is drawn with analysis of the three-dimensional current density and is further combined to the above-obtained cardiac contour cubic diagram, thereby enabling the estimation of the myocardial injury.

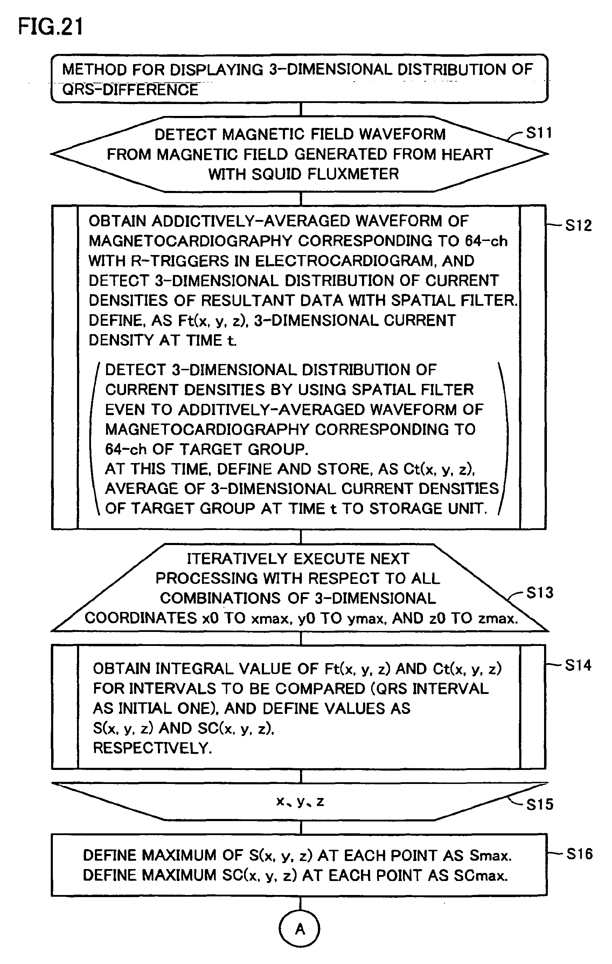

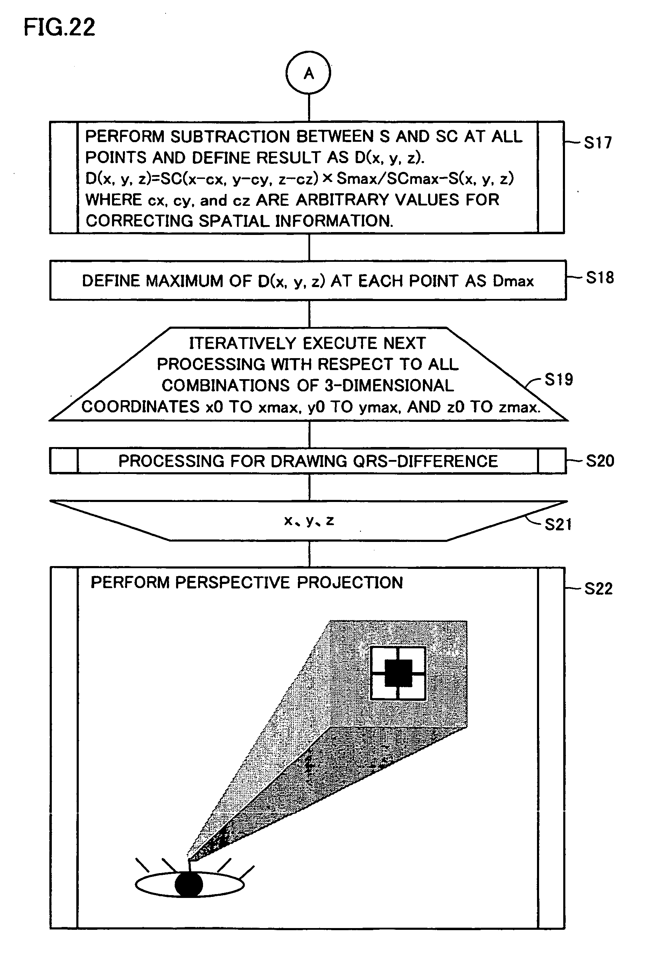

Figs. 21 and Fig. 22 are flowcharts showing a three-dimensional distribution display method of the QRS difference executed on software with the arithmetic operation unit 2 shown in Fig. 2.

Referring to Fig. 21, in step S 11, the cardiac magnetic-field of the subject is detected with the SQUID fluxmeter shown in Fig. 2, and the waveform of the cardiac magnetic-field is generated. Subsequently, in step S12, the additively-average waveform of the magnetocardiography signals (Figs. 4 and 5) corresponding to 64 channels of the subject is obtained with R triggers in the electrocardiogram obtained with the electrocardiograph 21 shown in Fig. 2 is obtained and the three-dimensional distribution of the current densities of the resultant data is detected with the spatial filter. Herein, the three-dimensional current density of the subject at the time t is defined as $Ft(x, y, z)$.

Especially, if the subject is a healthy individual forming a target group (e.g., healthy individuals of 30 members), the spatial filter is used even to the additively-average waveform of the magnetocardiography signals corresponding to the 64 channels of the subjects (healthy individuals), thereby detecting the three-dimensional current density distribution. Further, the average of the three-dimensional current densities of all subjects (healthy individuals) forming the target group at the time t is defined as $Ct(x, y, z)$, and is stored to the storage unit 22 shown in Fig. 2.

Subsequently, steps S 13, S 14, and S 15 indicate loop processing for obtaining the integral value of the three-dimensional distribution of the current densities. With respect to all combinations of three-dimensional coordinates x0 to xmax, y0 to ymax, and z0 to zmax shown in step S 13, the processing in step 14 is iteratively executed until closing the loop of x, y, and z in step S 15.

That is, in step S 14, for the intervals corresponding to the cardiac portion whose three-dimensional distribution of the current densities is to be compared between the target group (healthy individuals) and the subject, the integral values of the three-dimensional current density $Ft(x, y, z)$ of the subject at the time t and the three-dimensional average current density $Ct(x, y, z)$ of the target group stored in the storage unit 22 at the time t are individually defined as $S(x, y, z)$ and $SC(x, y, z)$.

Incidentally, the initial value of the interval to be compared is set between QRS intervals. The interval QRS corresponds to the ventricle of the cardiac contour. Therefore, the initial value of the QRS interval indicates the comparison in the distributions of the three-dimensional current densities of the ventricle between the subject and the average of the healthy individuals. By changing the interval to be compared, the distributions of the three-dimensional current densities of a portion other than the ventricle can be compared with each other.

Subsequently, in step S16, the maximum value of $S(x, y, z)$ at each point at the three-dimensional coordinate is defined as Smax, and the maximum value of $SC(x, y, z)$ at each point at the individual three-dimensional coordinates is defined as SCmax.

Subsequently, in step S17 in Fig. 22, at all points at the individual three-dimensional coordinate, subtraction is performed between the integral value S and the integral value SC by the following expression, and the result is defined as D(x, y, z).

$$D(x,\ y,\ z)=SC(x\text{-}cx,\ y\text{-}cy,\ z\text{-}cz)\times Smax/SCmax\text{-}S(x,\ y,\ z)$$

where cx, cy, and cz are arbitrary values for correcting the spatial information. That is, with respect to the measurement time of the subject and that of the healthy individual, although the measurement spaces are basically identical, the cardiac positions are displaced depending on the posture on the bed. Those are corrected with values cx, cy, and cz.

Subsequently, in step S 18, the maximum value of D(x, y, z) at each point at the individual three-dimensional coordinates is defined as Dmax.

Subsequently, steps S 19, S20, and S21 indicate loop processing for drawing the QRS difference. With respect to all combinations of three-dimensional coordinates x0 to xmax, y0 to ymax, and z0 to zmax shown in step S 19, processing for drawing the QRS-difference in step 20 is iteratively executed until closing the loop of x, y, and z in step S21.

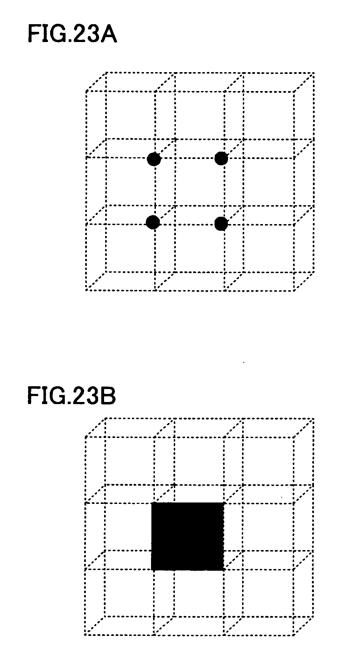

Figs. 23A and 23B are schematic diagrams conceptually showing the processing for drawing the QRS-difference in step S20 in Fig. 22. Referring to Fig. 23A, the points at the three-dimensional coordinates are drawn by linearly coloring with blue when D(x, y, z) is positive and with red when D(x, y, z) is negative. In Fig. 23A, two points on the top are colored with red and two points on the bottom are colored with blue. Incidentally, in Fig. 23A, for the purpose of convenience, the points are expressed with monochrome shading.

Next, the degrees of transparency (0.0 to 1.0) are added to the points by using the following expression with reference to Fig. 23B, and the interval between the points are subjected to color linear interpolation. That is, the degree of transparency is expressed by the following expression.

$$\text{The degree of transparency} = (|D\ (x,\ y,\ z)|\text{-threshold}) \div (Dmax\text{-threshold})$$

As mentioned above, the negative coordinate of the QRS difference D(x, y, z) is displayed with blue. In the case of the myocardial injury, the electromotive force of the myocardium is reduced. Therefore, the distribution of current densities is more reduced as compared with the average data of the target group (healthy individual) and the myocardial injury is displayed with blue. That is, by using the above expression of the degree of transparency, the shade of blue is determined depending on the value of the QRS difference D (x, y, z) to the maximum value Dmax of the QRS difference.

In the example in Fig. 23B, as the point is closer to the top of cube surrounded by four center points, the point is colored with red. Further, as the point is closer to the bottom of the cubic, the point is colored with blue. Therebetween, the point is linearly interpolated.

Next, perspective projection is performed in step S22 in Fig. 22 by using all the results of the processing for drawing the QRS-difference, repeated in steps S 19 to S21 in Fig. 22. The set of color display indicating the size of the QRS difference obtained as shown in Fig. 23B is subjected to the perspective projection, thereby obtaining image data on the QRS difference of the myocardium. The image data is restructured in the same space as that of the cardiac contour cubic diagram obtained in Figs. 7 to 15 with the arithmetic operation unit 2, and is displayed on the display of the display unit 4.

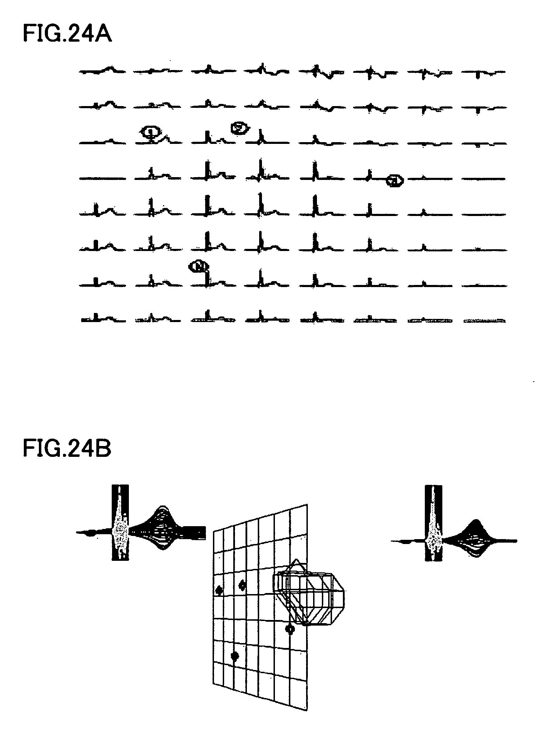

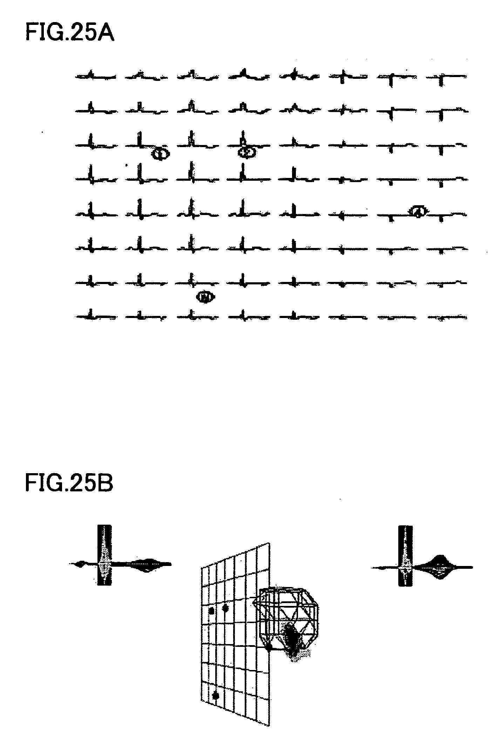

Figs. 24A and 24B are diagrams showing actual examples of the QRS difference of the healthy individual, and Figs. 25A and 25B are diagrams showing actual examples of the QRS difference of the patient. Figs. 24A and 25A show the signal waveforms of the magnetocardiography of the subject (the healthy individual in Fig. 24A and the patient with myocardial injury in Fig. 25A), and Figs. 24B and 25B show three-dimensional display of the corresponding QRS difference of the cardiac contour cubic diagram.

In Fig. 24B, the QRS difference between the healthy individuals in the heart is not identified.

On the other hand, in Fig. 25B, in the case of the myocardial injury (back side wall) such as the cardiac infarction portion, the QRS difference is displayed with blue, the reduction of the distribution of current densities, that is, the reduction of the electromotive force (myocardial injury) is indicated. In the restructured image shown in Figs. 24B and 25B, the blue density is replaced with the monochrome gradation density and the resultant data is displayed.

As mentioned above, according to the first embodiment of the present invention, the three-dimensional stereoscopic display of the QRS difference relatively-displaying the myocardial injury is obtained and this is reconstructed to the additionally-structured cardiac contour cubic diagram, thereby enabling absolute three-dimensional spatial display of the myocardial injury of the heart. Further, the localization of the myocardial injury can be determined in the diagnosis of the cardiac disease in the hospital or emergency room.

(Second embodiment)

According to the second embodiment of the present invention, the T-wave vector of the magnetocardiography can be three-dimensionally displayed, thereby enabling the determination of the three-dimensional spatial localization of the myocardial injury. Hereinbelow, the principle according to the second embodiment of the present invention will be described.

Referring back to Fig. 1, the actual waveform of the cardiac magnetic-field in (A) includes the T waves. As mentioned above, the T waves reflect the repolarization of the myocardium (particularly, the direction of repolarization). In the case of the healthy individual, the current vector of the QRS waves and the current vector of the T waves are in the same direction (approximately 45 degrees at the average of the healthy individual).

On the other hand, if the myocardium is damaged, the current vector of the T waves variously changes and, particularly, at the infarcted myocardium, it is just in the opposite direction (generally, negative 180 degrees). Therefore, the three-dimensional distribution of the current densities is obtained from the portion corresponding to the T waves of the magnetocardiography signal, and the current vector angle of the T waves is estimated, thereby enabling the determination of the myocardial injury.

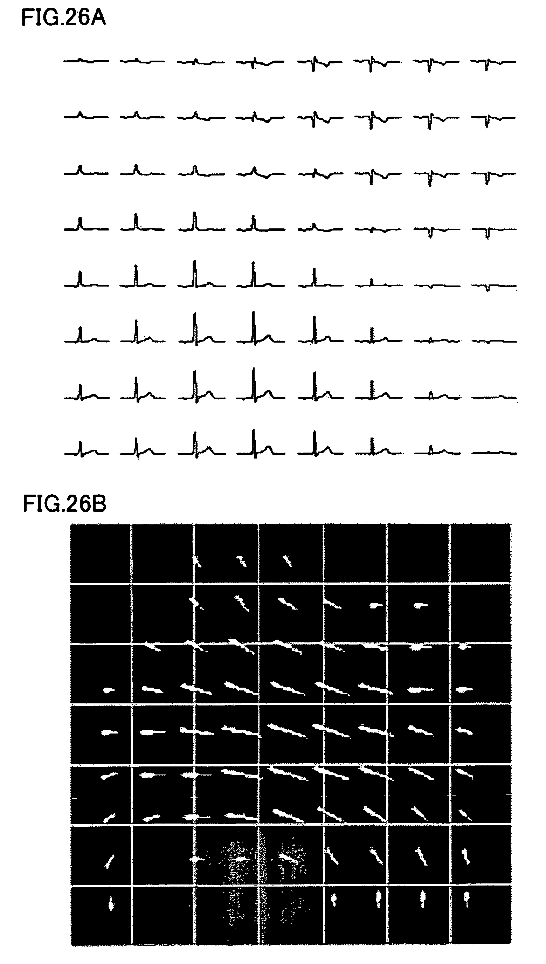

Figs. 26A and 26B are diagrams showing a relationship between the magnetocardiography signal and the current vector. Fig. 26A shows the 64-channel magnetocardiography waveform, including waveforms having the T waves of the channels as a peak and waveforms having those as a valley. Corresponding to the waveform of the cardiac magnetic-field, under the rule of a right screw, the current vector shown by an arrow in Fig. 26B is generated.

According to the second embodiment of the present invention, the three-dimensional current vector is obtained with the spatial filter from the portion corresponding to the T waves of the magnetocardiography signal of the subject. Further, the spatial distribution of the myocardial injury can be expressed by displaying operation in accordance with the current-vector angle obtained from a ratio of the x component and the y component of the current vector on the xy plane (displaying the direction of the current vector with the color).

However, only acquisition of the angle of the three-dimensional current vector can result in relatively determining the three-dimensional spatial localization of the myocardial injury of the heart, and the absolute three-dimensional spatial localization of the heart cannot be determined.

According to the second embodiment of the present invention, the cardiac contour can be drawn from the three-dimensional distribution of the current densities in the myocardium of the subject obtained from the measurement of the cardiac magnetic-field, and the angle of the current vector of the subject at the T waves is restructured in the same space of the drawn cardiac contour cubic diagram, thereby determining the absolute spatial localization of the myocardial injury on the three dimension of the heart of the subject.

Hereinbelow, a description will be given of the specific structure and operation realized according to the second embodiment of the present invention.

The hardware structure according to the second embodiment of the present invention is identical to the structure shown in Fig. 2 according to the first embodiment. Thus, a description thereof is omitted.

First, the arithmetic operation unit 2 shown in Fig. 2 executes the structuring method of the cardiac contour cubic diagram with reference to Figs. 7 to 15, thereby obtaining a cardiac contour cubic diagram shown in Fig. 19. The process has been described in detail and is not repeated here.

Next, the arithmetic operation unit 2 performs processing for restructuring the three-dimensional current density of the above-obtained cardiac contour cubic diagram.

That is, according to the second embodiment of the present invention, the T-wave vector (particularly, angle of the current vector) is drawn with the color by the analysis of the three-dimensional current density, and is combined to the above-obtained cardiac contour cubic diagram, thereby estimating the myocardial injury.

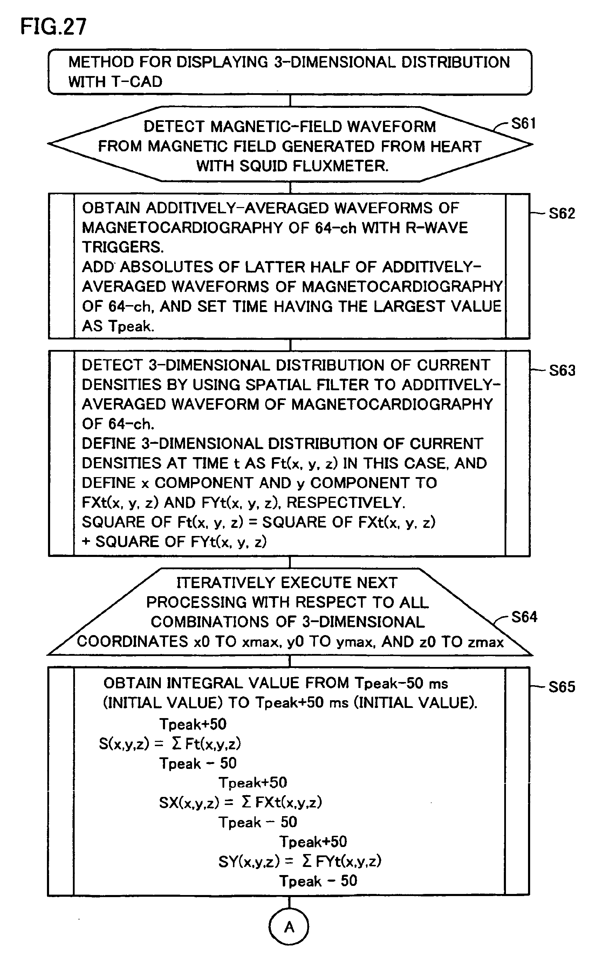

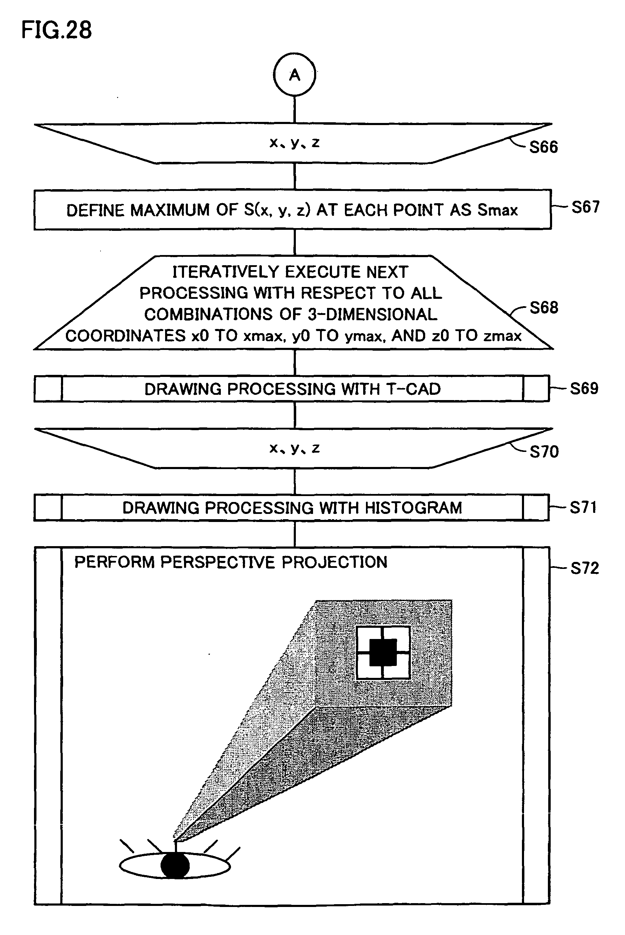

Figs. 27 and 28 are flowcharts of the three-dimensional distribution display method of the T-wave vector (hereinafter, referred to as a T-CAD method), executed on software by the arithmetic operation unit 2 shown in Fig. 2.

Referring to Fig. 27, in step S61, the cardiac magnetic-field of the subject is detected with the SQUID fluxmeter shown in Fig. 2 and the waveform of the cardiac magnetic-field is generated.

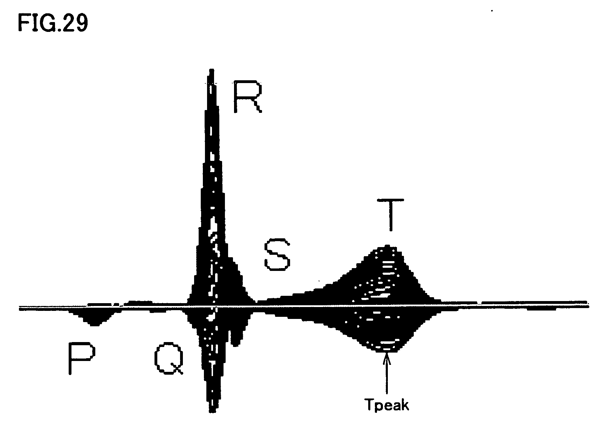

Next, in step S62, with R-wave triggers of the electrocardiogram obtained with the electrocardiograph 21 in Fig. 2, the magnetocardiography signals (in Figs. 4 and 5) of the 64 channels of the subject are additively averaged, thereby obtaining additive average waveforms shown in Fig. 29. Of the additive average waveforms shown in Fig. 29, a time at which the addition value of the latter half is maximum, i.e., a time of the top of a gentle peak (the T waves) is set as Tpeak.

Next, in step S63, the spatial filter is applied to the additive average waveform of the magnetocardiography signals of the 64 channels obtained in step S62, and the three-dimensional distribution of the current densities is detected. Herein, reference numeral Ft(x, y, z) denotes the three-dimensional current density of the subject at the time t. Further, FXt(x, y, z) denotes the x component and FYt(x, y, z) denotes the y component. In this case, the following relationship is established.

That is, the square of Ft(x, y, z) corresponds to the addition of the square of FXt(x, y, z) and the square of FYt(x, y, z).

Next, steps S64, S65, and S66 indicate loop processing for obtaining the integral value of the three-dimensional distribution of the current densities. With respect to all combinations of three-dimensional coordinates x0 to xmax, y0 to ymax, and z0 to zmax shown in step S64, processing in step 65 is iteratively executed until closing the loop of x, y, and z in step S66.

That is, in step S65, for the interval corresponding to the T waves, that is, for a period from Tpeak-50 ms to Tpeak+50 ms with Tpeak as center, the integral values of the three-dimensional current density Ft(x, y, z), x component FXt(x, y, z), and y component FYt(x, y, z) of the subject at the time t are obtained, thereby setting time as S(x, y, z), SX (x, y, z), and SY(x, y, z). It is noted that 50 ms is an initial value and an adjustable value.

Next, in step S67, reference numeral Smax denotes the maximum value of S(x, y, z) at each point at the individual three-dimensional coordinates.

Next, steps S68, S69, and S70 indicate loop processing for drawing the three-dimensional distribution display of the T-wave vector (T-CAD). With respect to all combinations of three-dimensional coordinates x0 to xmax, y0 to ymax, and z0 to zmax shown in step S68, processing for drawing the distribution of the T-wave vectors in step 69 is executed until closing the loop of x, y, and z in step S70.

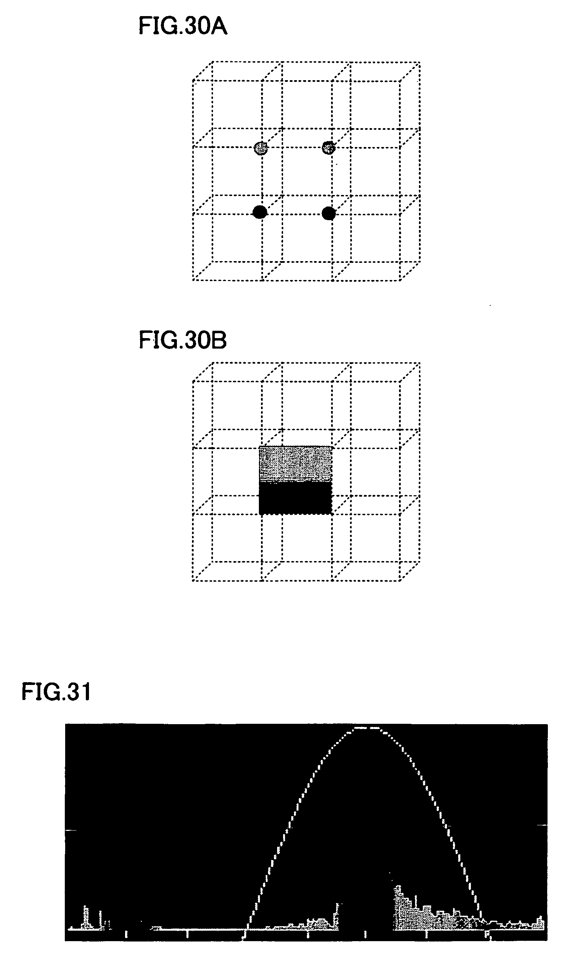

Figs. 30A and 30B are schematic diagrams conceptually showing the processing for drawing the distribution of the T-wave vectors in step S69 in Fig. 28. Referring to Fig. 30A, the angle of the current vector of the T waves is calculated by a ratio of the x component and y component of the current vector at each point at the individual three-dimensional coordinates with the following expression.

$$\text{arctan} \left( SY(x, y, z) \div SX(x, y, z) \right)$$

Herein, it is assumed that red corresponds to -135 degrees, green corresponds to -45 degrees, and blue corresponds to 45 degrees, and the point is drawn by linearly coloring in accordance with the angle of the current vector of the T waves. Referring to Fig. 30A, two points on the top are colored with thin blue, and two points on the bottom are colored with deep blue. It is noted that the point is expressed with monochrome density in Fig. 30A for the purpose of a convenience.

Next, referring to Fig. 30B, the degree of transparency (0:0 to 1.0) based on the following expression is added to the point in accordance with the size of the current vector of the T waves, and the interval between the points is subjected to color linear interpolation. That is, the degree of transparency is expressed by the following expression.

The degree of transparency = (S(x, y, z)-threshold) ÷ (Smax-threshold)

In the example in Fig. 30B, as the point is closer to the top of a cube surrounded by central four points, the blue is thinner. As the point is closer to the bottom, the blue is deeper. The interval between the points is linearly interpolated.

Next, in step S71, as shown in Fig. 31, a histogram obtained by layering S(x, y, z) as the size of the current vector to the angles (0 to 360 degrees) of the current vector is displayed. The histogram in Fig. 31 indicates the distribution of the T-wave vectors and the healthy individual indicates one peak with 45 degrees as center.

According to the second embodiment of the present invention, the T-wave vector of the healthy individual is indicated with blue (45 degrees) and the T-wave vector of the myocardial injury is indicated with red (-180 degrees).

Next, in step S72 in Fig. 28, the perspective projection is performed by adding all the results of the processing for drawing the distribution of the T-wave vectors iteratively-repeated in steps S68 to S70 in Fig. 28. The set of color display indicating the direction of the T-wave vector as obtained in Fig. 30B is subjected to the perspective projection, thereby obtaining the image data of the three-dimensional distribution of the T-wave vectors of the myocardium. The image data is restructured by the arithmetic operation unit 2 on the same space as that of the cardiac contour cubic diagram obtained in the processing in Figs. 7 to 15, and is displayed on the display of the display unit 4.

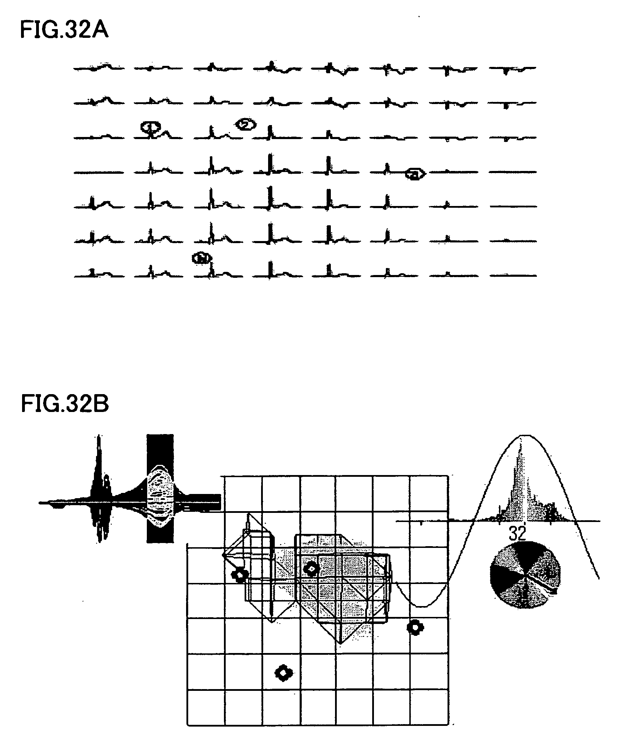

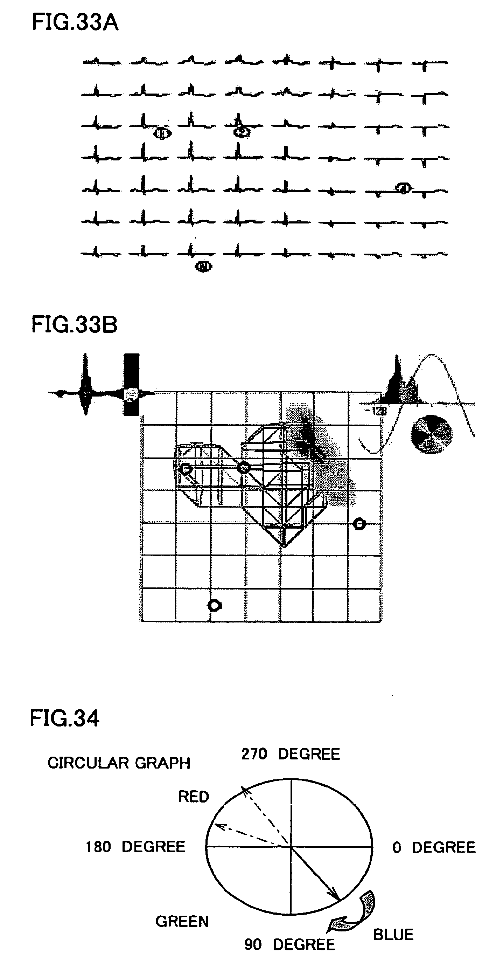

Figs. 32A and 32B are diagrams showing actual examples of the T-wave vector of the healthy individual. Figs. 33A and 33B are diagrams showing actual examples of the T-wave vector of the patient with myocardial injury. Figs. 32A and 33A show the waveforms of the magnetocardiography signal of the subject (the healthy individual in Fig. 32A and the patient with myocardial injury in Fig. 33A), and Figs. 32B and 33B show the three-dimensional display of the T-wave vector in the cardiac contour cubic diagram.

Fig. 34 is a diagram for illustrating the meaning of circular graphs in Figs. 32B and 33B. In the circular graph in Fig. 34, the T wave vectors are distributed near 45 degrees in the case of the healthy individual, as shown by a solid arrow (originally displayed on the image with blue). On the other hand, in the case in Fig. 33B, the T wave vectors are distributed near 200 to 220 degrees, as shown in by a broken arrow (originally displayed on the image with red).

In the case of the healthy individual in Fig. 32B, all the T-wave vectors are displayed with blue (corresponding to a vector angle of 45 degrees).

Further, referring to Fig. 33B, in the case of the myocardial injury (backside wall) such as the cardiac infarction, the T wave vector is displayed with red and green, and this indicates that the angle of the T-wave vector is at an abnormal area (corresponding to vector angles of 200 to 220 degrees) (indicating the myocardial injury). In the restructured image in Figs. 32B and 33B, the T wave vector is displayed with replacement of monochrome gradation.

As mentioned above, according to the second embodiment of the present invention, the three-dimensional stereoscopic display of the T-wave vector, relatively displaying the myocardial injury, is obtained. Further, the resultant data is restructured to the additionally structured cardiac contour cubic diagram, thereby enabling the absolute three-dimensional spatial display of the myocardial injury of the heart. Furthermore, it is possible to determine the localization of the myocardial injury in the diagnosis of the cardiac disease in the hospital or emergency treatment room.

(Third embodiment)

According to the second embodiment of the present invention, the RT-dispersion of the magnetocardiography can be three-dimensionally displayed, thereby enabling the determination of the three-dimensional spatial localization of the myocardial injury. Hereinbelow, the principle according to the third embodiment of the present invention will be described.

Referring again to Fig. 1, the actual waveform of the cardiac magnetic-field in (A) includes R waves and T waves. As mentioned above, the RT time serving as the interval between the R waves and the T waves reflects the repolarization time of the myocardium. Further, in the case of the healthy individual, the repolarization time is approximately equal, and corresponds to the time fluctuation of the repolarization between the maximum time and the minimum time, that is, the RT-dispersion is 20 ms to 40 ms.

On the other hand, if the myocardium is damaged, the RT-dispersion, serving as the time difference of the repolarization between the maximum time and the minimum time is a high value, i.e., 40 ms or more.

According to the third embodiment of the present invention, the distribution of the three-dimensional current density is obtained with the spatial filter from the portion corresponding to RT waves of the magnetocardiography of the subject. Further, the RT-dispersion on the three-dimension is calculated and the time distribution is stereoscopically displayed, thereby indicating the spatial distribution of the myocardial injury.

However, the obtaining of the time distribution of the RT-dispersion only can relatively determine the localization of the myocardial injury of the heart, and the absolute three-dimensional spatial localization of the heart cannot be determined.

According to the third embodiment of the present invention, it is possible to draw the cardiac contour from the three-dimensional distribution of the current densities from the myocardium of the subject, obtained with the measurement of the cardiac magnetic-field. Further, the time distribution of the RT-dispersion of the subject with the RT waves is restructured to the same space as that of the same subject of the drawn cardiac contour cubic diagram, thereby determining the spatial localization of the myocardial injury of the heart of the subject on the three-dimension.

Hereinbelow, a description will be given of the specific structure and operation for realizing the third embodiment of the present invention.

The hardware structure according to the third embodiment of the present invention is the same as the structure according to the first embodiment shown in Fig. 2 and a description thereof is thus omitted.

First, the arithmetic operation unit 2 shown in Fig. 2 executes the structuring method of the cardiac contour cubic diagram described with reference to Figs. 7 to 15, thereby obtaining the cardiac contour cubic diagram shown in Fig. 19. The process thereof has been already described in detail and is not thus repeated here.

Subsequently, the arithmetic operation unit 2 performs processing for restructuring the three-dimensional current density of the above-obtained cardiac contour cubic diagram.

That is, according to the third embodiment of the present invention, with the analysis of the three-dimensional current density, the time distribution of the RT-dispersion is drawn with colors and the resultant data is combined to the above-obtained cardiac contour cubic diagram, thereby enabling the estimation of the myocardial injury.

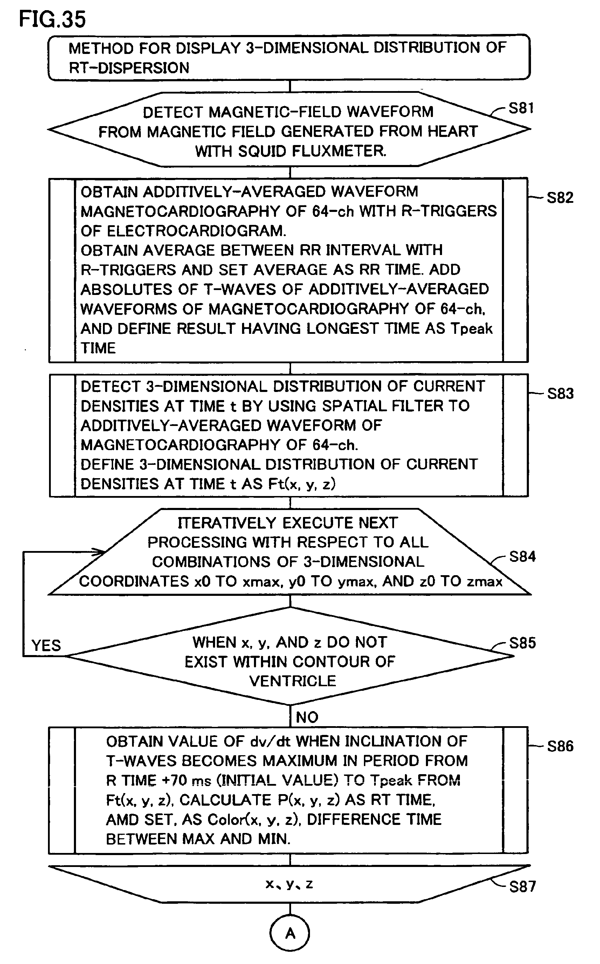

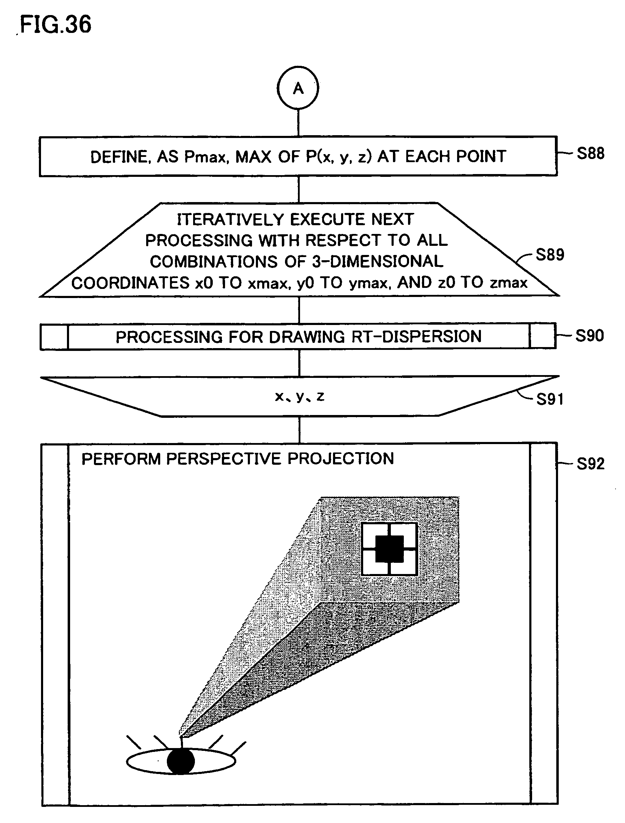

Figs. 35 and 36 are flowcharts showing the display method of the three-dimensional distribution of the RT-dispersion, executed on software with the arithmetic operation unit 2 shown in Fig. 2.

Referring to Fig. 35, in step S81, the cardiac magnetic-field of the subject is detected with the SQUID fluxmeter shown in Fig. 2, thereby generating the waveform of the cardiac magnetic-field.

Subsequently, in step S82, with R-wave triggers of the electrocardiogram obtained with the electrocardiograph 21 shown in Fig. 2, the magnetocardiography signals (shown in Figs. 4 and 5) of 64 channels of the subject are additively-averaged, thereby obtaining the additively-averaged waveform shown in Fig. 29. Further, with the R-wave triggers of the electrocardiogram, an average between the intervals of RR is obtained as an RR time.

Further, in the additively-averaged waveform shown in Fig. 29, a time at which the additive value at the latter half is maximum, i.e., time of the top of the T waves is obtained by viewing the waveform by the operator and is set as Tpeak

Subsequently, in step S83, the spatial filter is used to the additively-averaged waveform of the magnetocardiography signals of the 64 channels obtained in step S82, thereby detecting the three-dimensional distribution of the current densities. Herein, reference numeral Ft(x, y, z) denotes the three-dimensional current density of the subject at the time t.

Subsequently, steps S84 to S87 denotes loop processing for obtaining the RT-dispersion. The processing in step 86 is iteratively executed only to the three-dimensional coordinates that are determined to be in the cardiac contour (having the current density) in step S85 from all combinations of three-dimensional x0 to xmax, y0 to ymax, and z0 to zmax shown in step S84 until closing the loop of x, y, and z in step S87.

In step S86, in the interval corresponding to the QRS-T waves, that is, in the period from the R time +70 ms to the Tpeak, a value of dv/dt (value obtained by differentiating the current density by the time) when the inclination of the T waves becomes maximum (peak of the T waves) is obtained, and RT time as an accurate interval from the peak of the R waves to the peak of the T waves is obtained as P(x, y, z).

Further, the difference time between the maximum value and the minimum value of the calculated RT time P(x, y, z) is set as Color(x, y, z). It is noted that 70 ms is an initial value and is an adjustable value.

Subsequently, in step S88, the maximum value of P(x, y, z) at each point at the three-dimensional coordinates is set as Pmax.

Subsequently, steps S89, S90, and S91 indicate loop processing for drawing the RT-dispersion. The processing for drawing the RT-dispersion in step 90 is iteratively executed for all combinations of three-dimensional x0 to xmax, y0 to ymax, and z0 to zmax shown in step S89 until closing the loop of x, y, and z in step S91.

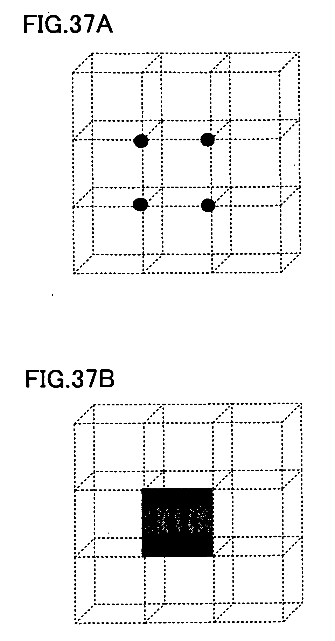

Figs. 37A and 37B are schematic diagrams conceptually showing the processing for drawing the RT-dispersion in step S90 in Fig. 36. Referring to Fig. 37A, the RT-dispersion is calculated at each point at each of the three-dimensional coordinates by the following expression.

That is, since the RT time changes depending on the heart rate, the RT-dispersion is corrected in accordance with the heart rate (the square root of the RR interval time) as shown by the following expression.

$$\text{(Color}(x, y, z) - \text{RT time}) \div \text{(square root of RR interval time)}$$

Herein, the blue is set as 0, the violet is set as 50, and the red is set as 100 and the linear coloring is performed in accordance with the RT-dispersion and the RT-dispersion is drawn. In Fig. 37A, two points on the top are colored with the red and two points on the bottom are colored with the blue. It is noted that the points are expressed with the monochrome shading in Fig. 37A for the purpose of a convenience.

Subsequently, referring to Fig. 37B, the degree of transparency (0.0 to 1.0) based on the following expression is added to points in accordance with the size of the RT-dispersion, and the interval between the points is linearly interpolated with colors. That is, the degree of transparency is expressed by the following expression.

$$\text{The degree of transparency} = (P(x, y, z) - \text{threshold}) \div (Pmax - \text{threshold})$$

In the example in Fig. 37B, as the point is closer to the top of a cube surrounded by central four points, the color becomes red and, as the point is closer to the bottom thereof, the color becomes blue. The interval between the points is linearly interpolated.

Subsequently, the perspective projection is performed in step S92 in Fig. 36 by using all results of the processing for drawing the RT-dispersion, repeated in steps S89 to S91 in Fig. 36. By the perspective projection of the set of color display indicating the RT-dispersion as obtained in Fig. 37B, the image data on the three-dimensional distribution of the RT-dispersion of the myocardium can be obtained, the arithmetic operation unit 2 restructures the image data in the same space as that of the cardiac contour cubic diagram obtained by the processing in Figs. 7 to 15, and the data is displayed on the display of the display unit 4.

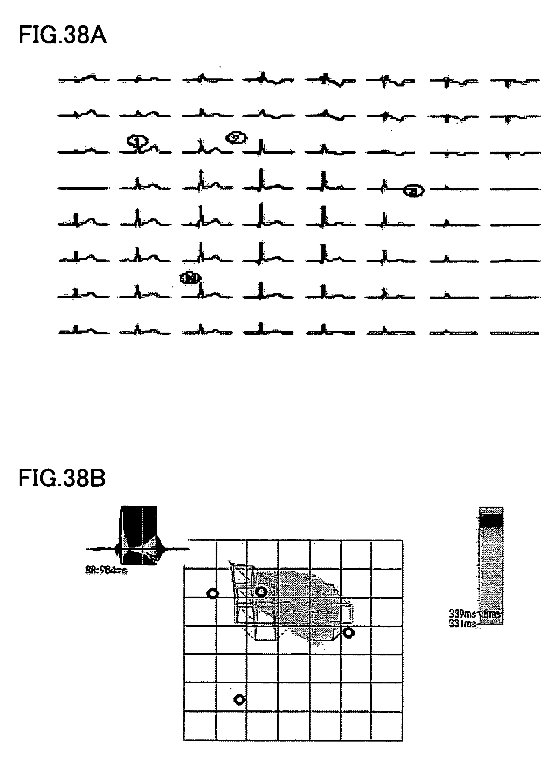

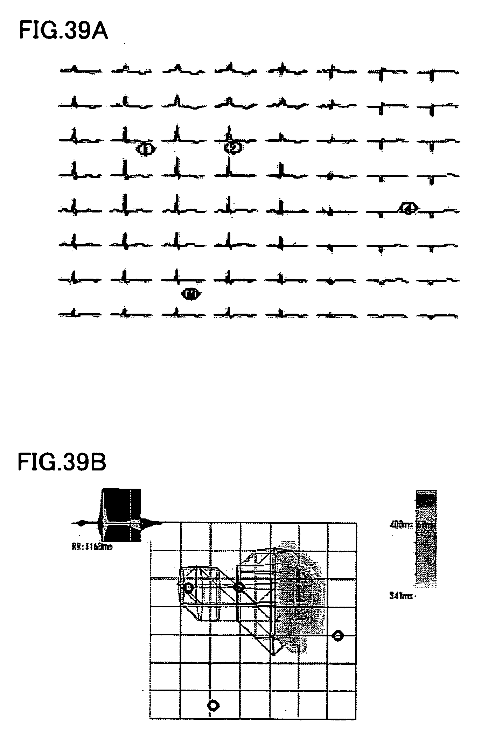

Figs. 38A and 38B are diagrams showing actual examples of the RT-dispersion of the healthy individual, Figs. 39A and 39B are diagrams showing actual examples of the RT-dispersion of the patient with myocardial injury. Figs. 38A and 39A show waveforms of the magnetocardiography signals of the subject (the healthy individual in Fig. 38A and the patient with myocardial injury in Fig. 39A). Figs. 38B and 39B show the corresponding three-dimensional display of the RT-dispersion of the cardiac contour cubic diagram.

Longitudinal graphs shown in Figs. 38B and 39B indicate the time distribution of the RT-dispersion (minimum 341 ms to maximum 408 ms). In the case of the healthy individual, the RT-dispersion is distributed within 38 ms (originally displayed with blue on the image). On the other hand, in the case shown in Fig. 39B, the RT-dispersion is distributed within 67 ms, that is, large (originally displayed with pink on the image).

In the case of the healthy individual shown in Fig. 38B, all the RT-dispersions are displayed with blue.

On the other hand, in the case shown in Fig. 39B, the myocardial injury (left room side wall) such as the cardiac infarction portion is displayed with pink, indicating that the RT-dispersion exists in the abnormal area (indicating the myocardial injury). In the restructured image shown in Figs. 38B and 39, the point is displayed with replacement of the monochrome gradation.

As mentioned above, according to the third embodiment of the present invention, the three-dimensional stereoscopic display of the RT-dispersion, relatively displaying the myocardial injury is obtained, and the resultant data is restructured to the additionally-structured cardiac contour cubic diagram. Thus, the absolute three-dimensional spatial display of the myocardial injury of the heart is possible and the localization of the myocardial injury in the diagnosis of the cardiac disease in the hospital or emergency treatment room can be determined.

(Fourth embodiment)

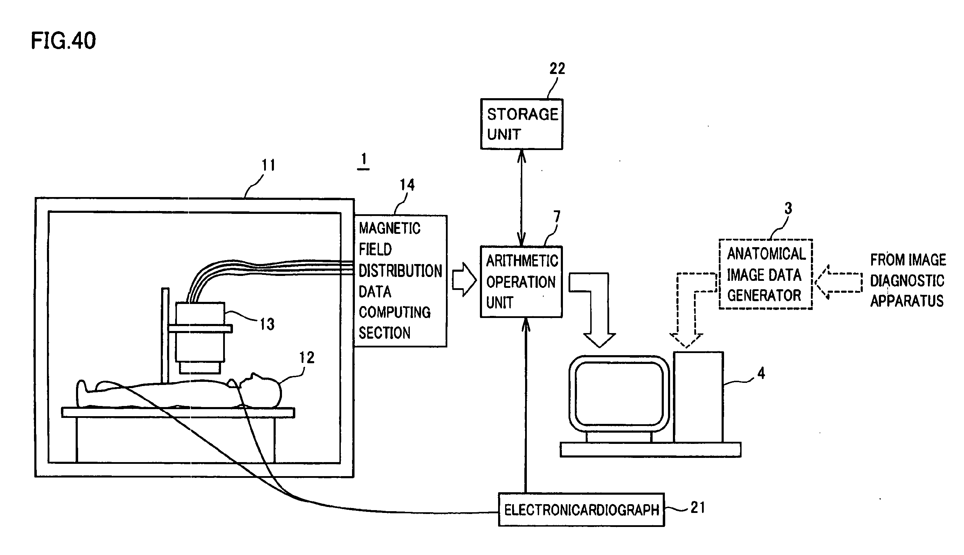

Fig. 40 is a block diagram showing the structure of the cardiac magnetic-field diagnostic apparatus according to the fourth embodiment of the present invention. According to the fourth embodiment, as shown in Fig. 40, the following points are different from the cardiac magnetic-field diagnostic apparatus according to the first embodiment shown in Fig. 2, and common portions are not described.

That is, according to the fourth embodiment, referring to Fig. 40, the magnetic field generator 5 and the coil 6 according to the first embodiment are not used, and a arithmetic operation unit 7 is provided in place of the arithmetic operation unit 2 according to the first embodiment.

Similarly to the arithmetic operation unit 2 shown in Fig. 2, the arithmetic operation unit 7 generates time-series data indicating the three-dimensional current density distribution of the heart as an analysis target from the data on the distribution of magnetic field generated by the magnetic-field distribution measurement device 1, and further generates a cardiac magnetic-field integral cubic diagram with the processing in Figs. 7 to 9, that is, the image data on the cardiac contour cubic diagram. Thereafter, the arithmetic operation unit 7 according to the fourth embodiment performs processing for structuring an excitation propagating locus of the above-obtained cardiac contour cubic diagram.

That is, according to the fourth embodiment of the present invention, with the above-mentioned analysis of the three-dimensional current density, the locus of the time-course excitation propagating locus of the impulse conducting system of the atrium and ventricle is drawn and is combinedto the additionally-obtained cardiac contour cubic diagram, thereby enabling the estimation of the signal source of various arrhythmia.

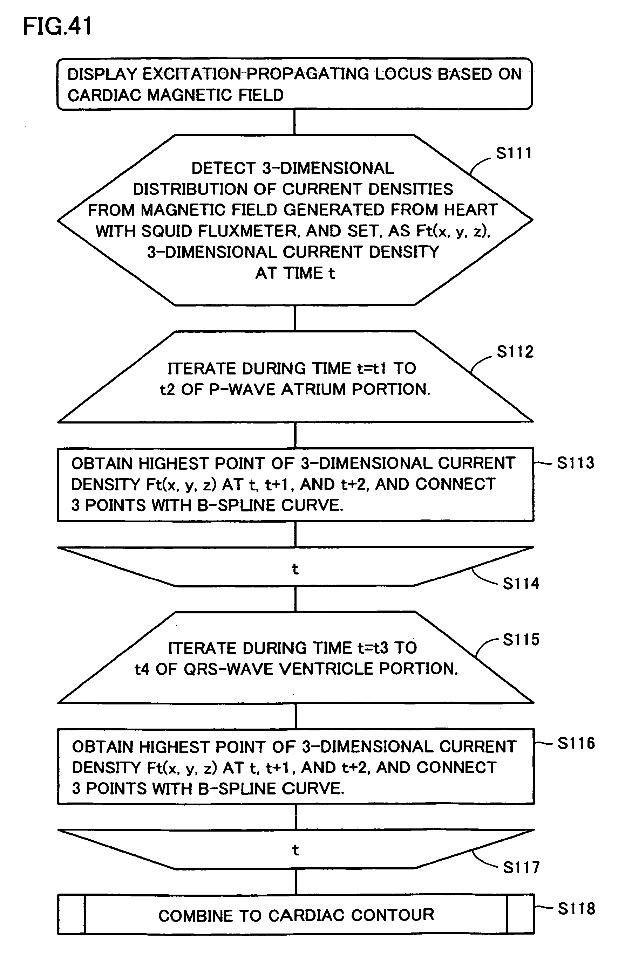

Fig. 41 is a flowchart of a structuring method of the excitation propagating locus executed on software with the arithmetic operation unit 7 shown in Fig. 40. In particular, the former-half steps S 111 to S 114 corresponds to a flowchart showing processing of the excitation propagating locus of the atrium among them.

Referring to Fig. 41, in step S111 according to the method with the spatial filter as described above with reference to Fig. 6, the three-dimensional current density is calculated from the distribution of the cardiac magnetic-field detected by the SQUID fluxmeter shown in Fig. 3. Herein, reference numeral Ft (x, y, z) denotes the three-dimensional current density of the three-dimensional coordinates x, y, and z of the subject the chest calculated at the time t. It is noted that data between the vertexes of the three-dimensional current density is subjected to linear interpolation.

Subsequently, steps S 112, S 113, and S 114 indicate loop processing for drawing the excitation propagating locus of the atrium portion of the heart. In step S 112, during a period from times t 1 to t2 of a P-wave atrium portion measured by the electrocardiograph 21 shown in Fig. 40, the processing for drawing the excitation propagating locus of the atrium in step S 113 is iteratively executed until closing the loop with respect to t in step S 114.

Subsequently, steps S 115 to S 117 indicate loop processing for drawing the excitation propagating locus of the ventricle, executed subsequently to the processing in steps S111 to 114. Steps S 115 to S 117 are identical to the processing in steps S 112 to S 114, except for a point that the processing period corresponds to times t3 to t4 of the QRS-waves ventricle portion, measured by the electrocardiograph 21 and common portions are not therefore described.

Subsequently, the common processing in steps S113 and S116 will be described. For example, at the time of the P-wave atrium portion in step S 13, the strongest points of Ft (x, y, z) at each timing are connected by selecting three timings t, t+1, and t+2 during the period t1 to t2.

At this time, the line obtained by simply connecting the three points with straight lines is zigzag. Therefore, the three points are connected with a well-known B-spline curve. The B-spline curve indicates a median point of a triangle reflexively-obtained (refer to, e.g., http://musashi.or.tv.doc/doc2.htm).

As mentioned above, the three strongest points of Ft(x, y, z) at each of the timings t, t+1, and t+2 are connected with the B-spline curve, the three strongest points of Ft(x, y, z) at each of the timings t+1, t+2, and t+3 shifted in the period from t1 to t2 are connected with the B-spline curve, and the three strongest points of Ft(x, y, z) at each of the timings t+2, t+3, and t+4 shifted in the period from t1 to t2 are connected with the B-spline curve.

The above-mentioned loop processing is iterated during the period from t1 to t2 of the P wave, thereby obtaining a line for connecting the strongest points of the three-dimensional current density.

At the time of the QRS waves ventricle portion in step S16, during a period from t3 to t4, similarly, the strongest points of Ft(x, y, z) at three timings t, t+1, and t+2 are connected by selecting the timings. The following processing is identical to that in step S13.

By drawing the locus of the strongest points of the current density, it is possible to draw the time-course excitation propagating locus of the impulse conducting system of the atrium and the ventricle.

According to the fourth embodiment of the present invention, the above-mentioned magnetic-field integral cubic diagram obtained according to the first embodiment, that is, the cardiac contour cubic diagram and the excitation propagating iocus are restructured. Thus, it is possible to three-dimensionally display the excitation propagating locus from the atrium and the ventricle to the Purkinje fiber through the sinus node and the atrioventricular node.

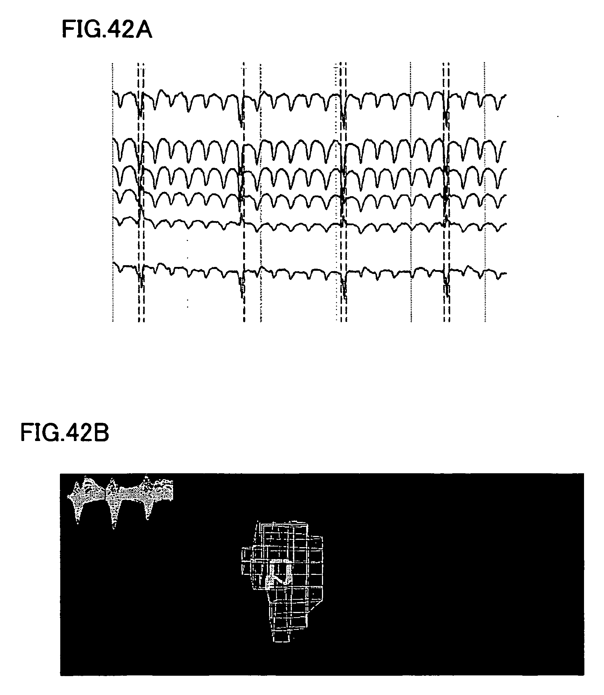

Fig. 42A is a waveform of a magnetocardiography of atrial flutter as one example of the arrhythmia. Fig. 42B is a diagram showing the combination of an excitation circulating circuit, that is re-entry circuit (figure drawn by a thick line in the diagram) in the atrium of the atrial flutter, obtained according to the second embodiment, in the cardiac contour cubic diagram (figure drawn by a thin line in the diagram) obtained according to the method of the present invention the first embodiment. In the example, the identification is possible in the cardiac contour cubic diagram of the re-entry circuit of the atrial flutter. However, according to the second embodiment, it is possible to estimate the signal sources of various arrhythmia such as WPW syndrome and atrial fibrillation in addition to the atrial flutter.

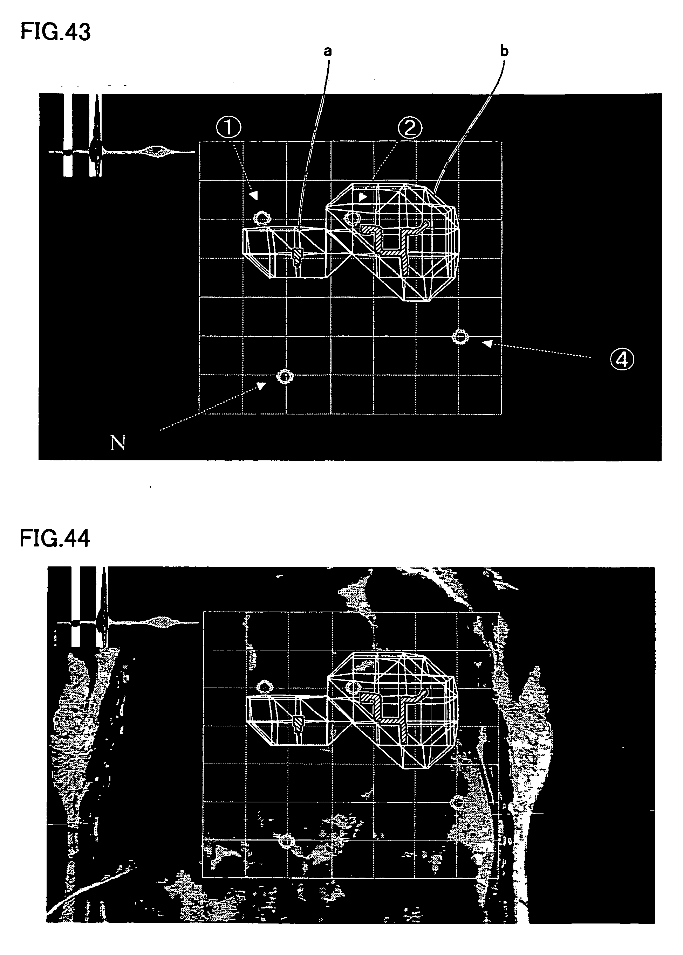

Fig. 43 is a diagram showing the restructure of the excitation propagating locus in addition to the spatial recognition of the cardiac contour cubic diagram according to the first embodiment. Thus, the excitation propagating locus can be anatomically and spatially identified with accuracy.

The excitation propagating locus can be structured and it can thus be easily combined to the anatomical image data, e.g., MRI or CT as needed. Referring to Fig. 40, as needed, an anatomical image data generator 3 shown by a broken line receives slice image data of the chest of the same subject captured by another tomographic diagnostic apparatus with, e.g., MRI or X-ray CT.

The anatomical image data generator 3 generates data indicating three-dimensional anatomical image of the chest near the heart of the same subject, and sends the resultant data to another input of the display unit 4.

The display unit 4 shown in Fig. 40 overlays the image indicating the cardiac contour and the excitation propagating locus formed based on the data on the cardiac magnetic-field integral cubic diagram from the arithmetic operation unit 7 to the three-dimensional anatomical image of anatomical image of the chest of the subject formed based on the data from the anatomical image data generator 3 and the resultant data is displayed.

Fig. 44 is a cubic diagram of the cardiac contour shown in Fig. 43 and showing the combination of the excitation propagating locus and the MRI image. According to the spatial recognizing method of the first embodiment, as the same points as the above four coils on the body surface of the same subject in the MRI measurement are marked with a marker, thereby enabling accurate combination to the cardiac contour cubic diagram without spatial displacement.

As mentioned above, according to the fourth embodiment of the present invention, the cardiac magnetic-field integral cubic diagram is drawn as a cubic diagram of the cardiac contour from the distribution of the current densities of the myocardium calculated based on the noninvasive measurement of the cardiac magnetic-field, thereby structuring the excitation propagating locus of the heart.

In particular, the cardiac contour cubic diagram and excitation propagating locus of the same subject measured at the same time according to the same measurement method are restructured on the same space. Thus, the excitation propagating locus can be extremely accurately identified without the spatial displacement therebetween.

According to the first to fourth embodiments, the number of channels of the SQUID fluxmeter is 64 and, however, the present invention is not limited to this. Further, the number of coils attached to the body surface of the subject is not limited to 4.

Further, according to the first to fourth embodiments, the cardiac contour cubic diagram is obtained with the integral value of data on the three-dimensional current density. However, in place of this, the cardiac contour cubic diagram can be obtained with an integral value of three-dimensional energy density data. That is, if impedance of the living body is constant, the data on the current density is squared, thereby obtaining the data on the energy density. In the processing of the flowcharts shown in Figs. 7 to 9, in place of integral value of the data on the three-dimensional current density, the cardiac contour cubic diagram can be similarly obtained with an integral value of data on three-dimensional energy density obtained by further squaring the data on the three-dimensional current density. As a consequence, the same advantages as those according to the first to fourth embodiments can be obtained.

It should be considered that the embodiments disclosed herein are only examples in all points and do not limit the present invention. The range of the present invention is indicated not by the above description but by claims, and the entire modifications and changes should be included within the identical meaning and range of claims.

**Industrial Applicability**

According to the present invention, the accurate spatial recognition of the heart and the three-dimensional localization of the myocardial injury can be determined by non-invasive measurement of the cardiac magnetic-field with low burden to the patient, and is suitable to the field of image diagnostic apparatus using the measurement of the cardiac magnetic-field.

**Claims**

1.  A cardiac magnetic-field diagnostic apparatus for performing three-dimensional localization of a myocardial injury, comprising:

    cardiac magnetic-field distribution measuring means (1) that generates data on a two-dimensional distribution of a cardiac magnetic-field corresponding to a plurality of coordinates on the chest of a subject with contactless magnetic measurement of the plurality of co-ordinates;
    current-density data generating means (2) that generates data on a three-dimensional distribution of current densities of the myocardium of the subject on the basis of the generated data on the two-dimensional distribution of the cardiac magnetic-field; and
    myocardial injury data generating means (2) that generates data indicating the three-dimensional localization of a myocardial injury of the heart on the basis of the data on the three-dimensional distribution of the current densities,

    **characterized by**
    cardiac cubic diagram structuring means (2) that structures a cardiac magnetic-field integral cubic diagram indicating a cardiac contour on the basis of the data on the three-dimensional distribution of the current densities; and
    image restructuring means (2) that restructures the three-dimensional localization of the myocardial injury on the same space as that of the structured cardiac magnetic-field integral cubic diagram.

2.  The cardiac magnetic-field diagnostic apparatus according to Claim 1, wherein the myocardial injury data generating means comprises:

    difference calculating means that obtains the QRS difference between average data of pre-obtained data on a three-dimensional distribution of the current densities of QRS waves of a plurality of healthy individuals and data on a three-dimensional distribution of the current densities of QRS waves of the subject; and
    drawing data generating means that generates data for drawing the three-dimensional localization of the myocardial injury on the basis of the obtained QRS difference.

3.  The cardiac magnetic-field diagnostic apparatus according to Claim 1, wherein the myocardial injury data generating means comprises:

    vector angle calculating means that obtains an angle of a current vector from data on a three-dimensional distribution of the current densities of T waves of the subject; and
    drawing data generating means that generates data for drawing the three-dimensional localization of the myocardial injury on the basis of the obtained angle of the current vector of the T waves.

4.  The cardiac magnetic-field diagnostic apparatus according to Claim 1, wherein the myocardial injury data generating means comprises:

    time distribution calculating means that obtains an RT-dispersion, as a distribution of RT time, from data on a three-dimensional distribution of the current densities of QRS-T waves of the subject; and
    drawing data generating means that generates data for drawing the three-dimensional localization of the myocardial injury on the basis of the obtained RT-dispersion.

5.  The cardiac magnetic-field diagnostic apparatus according to Claim 1, wherein the cardiac cubic diagram structuring means comprises:

    integrating means that obtains an integral value for a predetermined period of data on the three-dimensional distribution of the current densities at the three-dimensional coordinates of the chest of the subject, or of data on three-dimensional energy density, obtained by squaring the data on the three-dimensional distribution of the current densities;

maximum-value determining means that obtains a maximum value of the integral values at the coordinates;

cube setting means that segments the three-dimensional coordinates of the chest into a plurality of sets of cubes;

threshold setting means that sets a threshold on the basis of the maximum value of the integral values; and

high/low determining means that determines whether the integral value at the coordinates corresponding to a vertex of the cube is higher or lower than the set threshold;

image generating means that generates, as the cardiac magnetic-field integral cubic diagram, an image displaying the high/low determination result of the integral value in the set of a plurality of cubes.

6. An operation method of a cardiac magnetic-field diagnostic apparatus for evaluating three-dimensional localization of a myocardial injury, comprising:

   a step of generating data on a two-dimensional distribution of a cardiac magnetic-field corresponding to a plurality of coordinates of the chest of a subject with contactless magnetic measurement;

   a step of generating data on a three-dimensional distribution of current densities of the myocardium of the subject on the basis of the generated data on the two-dimensional distribution of the cardiac magnetic-field; and

   a step of generating data indicating three-dimensional localization of the myocardial injury of the heart on the basis of the data on the three-dimensional distribution of the current densities,

   **characterized by**
   a step of structuring a cardiac magnetic-field integral cubic diagram indicating a cardiac contour on the basis of the data on the three-dimensional distribution of the current densities; and

   a step of restructuring the three-dimensional localization of the myocardial injury on the same space as that of the structured cardiac magnetic-field integral cubic diagram.

7. The operation method of the cardiac magnetic-field diagnostic apparatus according to Claim 6, wherein the step of generating the data indicating the three-dimensional localization of the myocardial injury comprises:

   a step of obtaining the QRS difference between average data of pre-obtained data on the three-dimensional distribution of the current densities of QRS waves of a plurality of healthy individuals and data on the three-dimensional distribution of the current densities of the QRS waves of the subject; and

   a step of generating data for drawing the three-dimensional localization of the myocardial injury on the basis of the obtained QRS difference.

8. The operation method of the cardiac magnetic-field diagnostic apparatus according to Claim 6, wherein the step of generating the data indicating the three-dimensional localization of the myocardial injury comprises:

   a step of obtaining an angle of a current vector from the data on the three-dimensional distribution of the current densities of T waves of the subject; and

   a step of generating data for drawing the three-dimensional localization of the myocardial injury on the basis of the obtained angle of the current vector of the T waves.

9. The operation method of the cardiac magnetic-field diagnostic apparatus according to Claim 6, wherein the step of generating the data indicating the three-dimensional localization of the myocardial injury comprises:

   a step of obtaining RT-dispersion, as distribution of RT time from data on three-dimensional distribution of the current densities of QRS-T waves of the subject; and

   a step of generating data for drawing the three-dimensional localization of the myocardial injury on the basis of the obtained RT-dispersion.

10. The operation method of the cardiac magnetic-field diagnostic apparatus according to Claim 6, wherein the step of structuring the cardiac magnetic-field integral cubic diagram comprises:

   a step of obtaining an integral value for a predetermined period of the data on the three-dimensional distribution of the current densities of the chest of the subject at the three-dimensional coordinates, or of data on three-dimensional energy density, obtained by squaring the data on the three-dimensional distribution of the current densities;

a step of obtaining a maximum value of the integral values at the coordinates;

a step of segmenting the three-dimensional coordinates of the chest into a plurality of sets of cubes;

a step of setting a threshold on the basis of the maximum value of the integral values;

a step of determining whether the integral value at the coordinates corresponding to a vertex of the cube is higher or lower than the set threshold; and

a step of generating, as the cardiac magnetic-field integral cubic diagram, an image displaying the high/low determining result of the integral value in the set of the plurality of cubes.

**Patentansprüche**

1. Herzmagnetfelddiagnosegerät zum Durchführen einer dreidimensionalen Lokalisierung einer Herzmuskelbeschädigung, mit:

einer Herzmagnetfeldverteilungsmesseinrichtung (1), die Daten einer zweidimensionalen Verteilung eines Herzmagnetfelds entsprechend vielen Koordinaten an der Brust eines Subjekts durch kontaktlose, magnetische Messung der vielen Koordinaten erzeugt;

einer Stromdichtendatenerzeugungseinrichtung (2), die Daten einer dreidimensionalen Verteilung von Stromdichten des Herzmuskels des Subjekts auf der Grundlage der erzeugten Daten der zweidimensionalen Verteilung des Herzmagnetfelds erzeugt; und

einer Herzmuskelbeschädigungsdatenerzeugungseinrichtung (2), die Daten erzeugt, die die dreidimensionale Lokalisierung einer Herzmuskelbeschädigung des Herzens auf der Grundlage der Daten der dreidimensionalen Verteilung der Stromdichten angeben,

**gekennzeichnet durch**
eine kubische Herzdiagrammstrukturiereinrichtung (2), die ein integrales, kubisches Herzmagnetfelddiagramm strukturiert, das eine Herzkontur auf der Grundlage der Daten der dreidimensionalen Verteilung der Stromdichten angibt; und

eine Bildrestrukturiereinrichtung (2), die die dreidimensionale Lokalisierung der Herzmuskelbeschädigung in demselben Raum wie das strukturierte, integrale kubische Herzmagnetfelddiagramm restrukturiert.

2. Herzmagnetfelddiagnosegerät gemäß Anspruch 1, wobei die Herzmuskelbeschädigungsdatenerzeugungseinrichtung Folgendes aufweist:

eine Differenzberechnungseinrichtung, die die QRS-Differenz zwischen Durchschnittsdaten der im Voraus erhaltenen Daten einer dreidimensionalen Verteilung der Stromdichten von QRS-Wellen von vielen gesunden Individuen und Daten einer dreidimensionalen Verteilung der Stromdichten von QRS-Wellen des Subjekts erhält; und

eine Zeichnungsdatenerzeugungseinrichtung, die Daten zum Zeichnen der dreidimensionalen Lokalisierung der Herzmuskelbeschädigung auf der Grundlage der erhaltenen QRS-Differenz erzeugt.

3. Herzmagnetfelddiagnosegerät gemäß Anspruch 1, wobei die Herzmuskelbeschädigungsdatenerzeugungseinrichtung Folgendes aufweist:

eine Vektorwinkelberechnungseinrichtung, die einen Winkel eines Stromvektors aus Daten einer dreidimensionalen Verteilung der Stromdichten von T-Wellen des Subjekts erhält; und

eine Zeichnungsdatenerzeugungseinrichtung, die Daten zum Zeichnen der dreidimensionalen Lokalisierung der Herzmuskelbeschädigung auf der Grundlage des erhaltenen Winkels des Stromvektors der T-Wellen erzeugt.

4. Herzmagnetfelddiagnosegerät gemäß Anspruch 1, wobei die Herzmuskelbeschädigungsdatenerzeugungseinrichtung Folgendes aufweist:

eine Zeitverteilungsberechnungseinrichtung, die eine RT-Dispersion als eine Verteilung einer RT-Zeit aus Daten einer dreidimensionalen Verteilung der Stromdichten von QRS-T-Wellen des Subjekts erhält; und

eine Zeichnungsdatenerzeugungseinrichtung, die Daten zum Zeichnen der dreidimensionalen Lokalisierung der Herzmuskelbeschädigung auf der Grundlage der erhaltenen RT-Dispersion erzeugt.

5. Herzmagnetfelddiagnosegerät gemäß Anspruch 1, wobei die kubische Herzdiagrammstrukturiereinrichtung Folgendes aufweist:

eine Integrationseinrichtung, die einen Integralwert für eine vorbestimmte Datenperiode der dreidimensionalen Verteilung der Stromdichten an den dreidimensionalen Koordinaten der Brust des Subjekts oder von Daten einer dreidimensionalen Energiedichte erhält, die dadurch erhalten wird, dass die Daten der dreidimensionalen Verteilung der Stromdichten quadriert werden;

eine Maximalwertbestimmungseinrichtung, die einen Maximalwert der Integralwerte an den Koordinaten erhält;

eine kubische Festlegungseinrichtung, die die dreidimensionalen Koordinaten der Brust in viele Hexaedersätze segmentiert;

eine Schwellwertfestlegungseinrichtung, die einen Schwellwert auf der Grundlage des Maximalwerts der Integralwerte festlegt; und

eine High/Low-Bestimmungseinrichtung, die bestimmt, ob der Integralwert an den Koordinaten entsprechend einem Eckpunkt des Hexaeders größer oder kleiner ist als der festgelegte Schwellwert;

eine Bilderzeugungseinrichtung, die als das integrale, kubische Herzmagnetfelddiagramm ein Bild erzeugt, das das High/Low-Bestimmungsergebnis des Integralwerts in dem Satz von vielen Hexaedern anzeigt.

6. Betriebsverfahren eines Herzmagnetfelddiagnosegeräts zum Auswerten einer dreidimensionalen Lokalisierung einer Herzmuskelbeschädigung, mit:

einem Schritt zum Erzeugen von Daten einer zweidimensionalen Verteilung eines Herzmagnetfelds entsprechend vielen Koordinaten der Brust eines Subjekts durch kontaktlose, magnetische Messung;

einem Schritt zum Erzeugen von Daten einer dreidimensionalen Verteilung von Stromdichten des Herzmuskels des Subjekts auf der Grundlage der erzeugten Daten der zweidimensionalen Verteilung des Herzmagnetfelds; und

einem Schritt zum Erzeugen von Daten, die eine dreidimensionale Lokalisierung der Herzmuskelbeschädigung des Herzens auf der Grundlage der Daten der dreidimensionalen Verteilung der Stromdichten angeben, **gekennzeichnet durch**

einen Schritt zum Strukturieren eines integralen, kubischen Herzmagnetfelddiagramms, das eine Herzkontur auf der Grundlage der Daten der dreidimensionalen Verteilung der Stromdichten angibt; und

einen Schritt zum Restrukturieren der dreidimensionalen Lokalisierung der Herzmuskelbeschädigung in demselben Raum wie das strukturierte, integrale, kubische Herzmagnetfelddiagramm.

7. Betriebsverfahren des Herzmagnetfelddiagnosegeräts gemäß Anspruch 6, wobei der Schritt zum Erzeugen der Daten, die die dreidimensionale Lokalisierung der Herzmuskelbeschädigung angeben, Folgendes aufweist:

einen Schritt zum Erhalten der QRS-Differenz zwischen Durchschnittsdaten der im Voraus erhaltenen Daten der dreidimensionalen Verteilung der Stromdichten von QRS-Wellen von vielen gesunden Individuen und Daten der dreidimensionalen Verteilung der Stromdichten der QRS-Wellen des Subjekts; und

einen Schritt zum Erzeugen von Daten zum Zeichnen der dreidimensionalen Lokalisierung der Herzmuskelbeschädigung auf der Grundlage der erhaltenen QRS-Differenz.

8. Betriebsverfahren des Herzmagnetfelddiagnosegeräts gemäß Anspruch 6, wobei der Schritt zum Erzeugen der Daten, die die dreidimensionale Lokalisierung der Herzmuskelbeschädigung angeben, Folgendes aufweist:

einen Schritt zum Erhalten eines Winkels eines Stromvektors aus den Daten der dreidimensionalen Verteilung der Stromdichten von T-Wellen des Subjekts; und

einen Schritt zum Erzeugen von Daten zum Zeichnen der dreidimensionalen Lokalisierung der Herzmuskelbeschädigung auf der Grundlage des erhaltenen Winkels des Stromvektors der T-Wellen.

9. Betriebsverfahren des Herzmagnetfelddiagnosegeräts gemäß Anspruch 6, wobei der Schritt zum Erzeugen der Daten, die die dreidimensionale Lokalisierung der Herzmuskelbeschädigung angeben, Folgendes aufweist:

einen Schritt zum Erhalten einer RT-Dispersion als eine Verteilung einer RT-Zeit aus Daten einer dreidimensionalen Verteilung der Stromdichten von QRS-T-Wellen des Subjekts; und

einen Schritt zum Erzeugen von Daten zum Zeichnen der dreidimensionalen Lokalisierung der Herzmuskelbeschädigung auf der Grundlage der erhaltenen RT-Dispersion.

**10.** Betriebsverfahren des Herzmagnetfelddiagnosegeräts gemäß Anspruch 6, wobei der Schritt zum Strukturieren des integralen, kubischen Herzmagnetfelddiagramms Folgendes aufweist:

einen Schritt zum Erhalten eines Integralwerts für eine vorbestimmte Periode der Daten der dreidimensionalen Verteilung der Stromdichten der Brust des Subjekts an den dreidimensionalen Koordinaten oder von Daten einer dreidimensionalen Energiedichte, die dadurch erhalten wird, dass die Daten der dreidimensionalen Verteilung der Stromdichten quadriert werden;

einen Schritt zum Erhalten eines Maximalwerts der Integralwerte an den Koordinaten;

einen Schritt zum Segmentieren der dreidimensionalen Koordinaten der Brust in viele Hexaedersätze;

einen Schritt zum Festlegen eines Schwellwerts auf der Grundlage des Maximalwerts der Integralwerte;

einen Schritt zum Bestimmen dessen, ob der Integralwert an den Koordinaten entsprechend einem Eckpunkt des Hexaeders größer oder kleiner ist als der festgelegte Schwellwert; und

einen Schritt zum Erzeugen eines Bilds, das ein High/Low-Bestimmungsergebnis des Integralwerts in dem Satz der vielen Hexaedern angibt, und zwar als das integrale, kubische Herzmagnetfelddiagramm.

## Revendications

**1.** Appareil de diagnostic de champ magnétique cardiaque pour effectuer une localisation tridimensionnelle d'une blessure myocardique, comprenant :

des moyens de mesure de distribution de champ magnétique cardiaque (1) qui génèrent des données sur une distribution bidimensionnelle d'un champ magnétique cardiaque correspondant à une pluralité de coordonnées sur la poitrine d'un sujet avec une mesure magnétique sans contact de la pluralité de coordonnées ;

des moyens de génération de données de densité de courant (2) qui génèrent des données sur une distribution tridimensionnelle de densités de courant du myocarde du sujet sur la base des données générées sur la distribution bidimensionnelle du champ magnétique cardiaque ; et

des moyens de génération de données de blessure myocardique (2) qui génèrent des données indiquant la localisation tridimensionnelle d'une blessure myocardique du coeur sur la base des données sur la distribution tridimensionnelle des densités de courant,

**caractérisé par**

des moyens de structuration de diagramme cubique cardiaque (2) qui structurent un diagramme cubique intégral de champ magnétique cardiaque indiquant un contour cardiaque sur la base des données sur la distribution tridimensionnelle des densités de courant ; et

des moyens de restructuration d'image (2) qui restructurent la localisation tridimensionnelle de la blessure myocardique sur le même espace que celui du diagramme cubique intégral de champ magnétique cardiaque structuré.

**2.** Appareil de diagnostic de champ magnétique cardiaque selon la revendication 1, **caractérisé en ce que** les moyens de génération de données de blessure myocardique comprennent :

des moyens de calcul de différence qui obtiennent la différence QRS entre des données moyennes de données pré-obtenues sur une distribution tridimensionnelle des densités de courant d'ondes QRS d'une pluralité d'individus en bonne santé et des données sur une distribution tridimensionnelle des densités de courant d'ondes QRS du sujet ; et

des moyens de génération de données de dessin qui génèrent des données pour dessiner la localisation tridimensionnelle de la blessure myocardique sur la base de la différence QRS obtenue.

**3.** Appareil de diagnostic de champ magnétique cardiaque selon la revendication 1, **caractérisé en ce que** les moyens de génération de données de blessure myocardique comprennent :

des moyens de calcul d'angle de vecteur qui obtiennent un angle d'un vecteur de courant à partir de données sur une distribution tridimensionnelle des densités de courant d'ondes T du sujet ; et

des moyens de génération de données de dessin qui génèrent des données pour dessiner la localisation tridimensionnelle de la blessure myocardique sur la base de l'angle obtenu du vecteur de courant des ondes T.

**4.** Appareil de diagnostic de champ magnétique cardiaque selon la revendication 1, **caractérisé en ce que** les moyens de génération de données de blessure myocardique comprennent :

des moyens de calcul de distribution de temps qui obtiennent une dispersion RT, comme une distribution de temps RT, à partir de données sur une distribution tridimensionnelle des densités de courant d'ondes QRS-T du sujet ; et

des moyens de génération de données de dessin qui génèrent des données pour dessiner la localisation tridimensionnelle de la blessure myocardique sur la base de la dispersion RT obtenue.

5. Appareil de diagnostic de champ magnétique cardiaque selon la revendication 1, **caractérisé en ce que** les moyens de structuration de diagramme cubique cardiaque comprennent :

des moyens d'intégration qui obtiennent une valeur intégrale pour une période prédéterminée de données sur la distribution tridimensionnelle des densités de courant au niveau des coordonnées tridimensionnelles de la poitrine du sujet, ou de données sur une densité d'énergie tridimensionnelle, obtenues en élevant au carré les données sur la distribution tridimensionnelle des densités de courant ;

des moyens de détermination de valeur maximale qui obtiennent une valeur maximale des valeurs intégrales au niveau des coordonnées ;

des moyens d'établissement de cube qui segmentent les coordonnées tridimensionnelles de la poitrine en une pluralité d'ensembles de cubes ;

des moyens d'établissement de seuil qui établissent un seuil sur la base de la valeur maximale des valeurs intégrales ; et

des moyens de détermination haut/bas qui déterminent si la valeur intégrale au niveau des coordonnées correspondant à un sommet du cube est supérieure ou inférieure au seuil établi ;

des moyens de génération d'image qui génèrent, comme le diagramme cubique intégral de champ magnétique cardiaque, une image affichant le résultat de détermination haut/bas de la valeur intégrale dans l'ensemble d'une pluralité de cubes.

6. Procédé de fonctionnement d'un appareil de diagnostic de champ magnétique cardiaque pour évaluer une localisation tridimensionnelle d'une blessure myocardique, comprenant :

une étape consistant à générer des données sur une distribution bidimensionnelle d'un champ magnétique cardiaque correspondant à une pluralité de coordonnées de la poitrine d'un sujet avec une mesure magnétique sans contact ;

une étape consistant à générer des données sur une distribution tridimensionnelle de densités de courant du myocarde du sujet sur la base des données générées sur la distribution bidimensionnelle du champ magnétique cardiaque ; et

une étape consistant à générer des données indiquant la localisation tridimensionnelle de la blessure myocardique du coeur sur la base des données sur la distribution tridimensionnelle des densités de courant,

**caractérisé par**
une étape consistant à structurer un diagramme cubique intégral de champ magnétique cardiaque indiquant un contour cardiaque sur la base des données sur la distribution tridimensionnelle des densités de courant ; et
une étape consistant à restructurer la localisation tridimensionnelle de la blessure myocardique sur le même espace que celui du diagramme cubique intégral de champ magnétique cardiaque structuré.

7. Procédé de fonctionnement de l'appareil de diagnostic de champ magnétique cardiaque selon la revendication 6, **caractérisé en ce que** l'étape consistant à générer les données indiquant la localisation tridimensionnelle de la blessure myocardique comprend :

une étape consistant à obtenir la différence QRS entre des données moyennes de données pré-obtenues sur la distribution tridimensionnelle des densités de courant d'ondes QRS d'une pluralité d'individus en bonne santé et des données sur la distribution tridimensionnelle des densités de courant des ondes QRS du sujet ; et

une étape consistant à générer des données pour dessiner la localisation tridimensionnelle de la blessure myocardique sur la base de la différence QRS obtenue.

8. Procédé de fonctionnement de l'appareil de diagnostic de champ magnétique cardiaque selon la revendication 6, **caractérisé en ce que** l'étape consistant à générer les données indiquant la localisation tridimensionnelle de la blessure myocardique comprend :

une étape consistant à obtenir un angle d'un vecteur de courant à partir des données sur la distribution tridimensionnelle des densités de courant d'ondes T du sujet ; et

une étape consistant à générer des données pour dessiner la localisation tridimensionnelle de la blessure myocardique sur la base de l'angle obtenu du vecteur de courant des ondes T.

**9.** Procédé de fonctionnement de l'appareil de diagnostic de champ magnétique cardiaque selon la revendication 6, **caractérisé en ce que** l'étape consistant à générer les données indiquant la localisation tridimensionnelle de la blessure myocardique comprend :

une étape consistant à obtenir une dispersion RT, comme une distribution de temps RT, à partir de données sur une distribution tridimensionnelle des densités de courant d'ondes QRS-T du sujet ; et
une étape consistant à générer des données pour dessiner la localisation tridimensionnelle de la blessure myocardique sur la base de la dispersion RT obtenue.

**10.** Procédé de fonctionnement de l'appareil de diagnostic de champ magnétique cardiaque selon la revendication 6, **caractérisé en ce que** l'étape consistant à structurer le diagramme cubique intégral de champ magnétique cardiaque comprend :

une étape consistant à obtenir une valeur intégrale pour une période prédéterminée des données sur la distribution tridimensionnelle des densités de courant de la poitrine du sujet au niveau des coordonnées tridimensionnelles, ou de données sur une densité d'énergie tridimensionnelle, obtenues en élevant au carré les données sur la distribution tridimensionnelle des densités de courant ;
une étape consistant à obtenir une valeur maximale des valeurs intégrales au niveau des coordonnées ;
une étape consistant à segmenter les coordonnées tridimensionnelles de la poitrine en une pluralité d'ensembles de cubes ;
une étape consistant à établir un seuil sur la base de la valeur maximale des valeurs intégrales ;
une étape consistant à déterminer si la valeur intégrale au niveau des coordonnées correspondant à un sommet du cube est supérieure ou inférieure au seuil établi ; et
une étape consistant à générer, comme le diagramme cubique intégral de champ magnétique cardiaque, une image affichant le résultat de détermination haut/bas de la valeur intégrale dans l'ensemble de la pluralité de cubes.

FIG.1

RT DISPERSION

R

T-WAVE VECTOR

36ch
PQ=146(ms)
QRS=90(ms)
QT=400(ms)

(A)

T

P          Q     S

(B)

QRS DIFFERENCE

FIG.2

STORAGE
UNIT _22_

MAGNETIC FIELD
DISTRIBUTION
DATA
COMPUTING
SECTION _14_

ARITHMETIC
OPERATION
UNIT _2_

ANATOMICAL
IMAGE DATA
GENERATOR _3_

FROM IMAGE
DIAGNOSTIC
APPARATUS

MAGNETIC
FIELD
GENERATOR _5_

ELECTROCARDIOGRAPH _21_

4

EP 1 769 741 B1

FIG.3

SQUID FLUXMETER 15

COMPUTING SECTION 14

REF

V/I

+
−

∫ dt

LPF

notch 50/60Hz

ISO Amp

OSC 30~50kHz

~10kHz

LOW-TEMPERATURE SYSTEM IN DEWAR 13

NORMAL-TEMPERATURE SYSTEM IN MSR 11

EP 1 769 741 B1

FIG.4

25mm

RIGHT

175mm

LEFT

175mm

FIG.5

HEAD

RIGHT

LEFT

LEG

# FIG.6

(1)SET ANALYSIS TARGET
AS SET OF VOXELS

COMPONENT ORTHOGONAL TO PLANE IS
OMITTED IN MANY CASES BECAUSE
MAGNETIC-FIELD SENSOR IS ARRANGED ON
PLANE OF CHEST IN MEASUREMENT OF
CARDIAC MAGNETIC-FIELD IN MANY CASES

(2)ARRANGE VOXEL TO WHICH DISTRIBUTION
CURRENT OF ORTHOGONAL COMPONENTS
ON THE SAME COORDINATE

MAGNETIC-FIELD
SENSOR

EP 1 769 741 B1

# FIG.7

METHOD FOR OBTAINING CARDIAC CONTOUR BASED ON MAGNETIC FIELD OF HEART

DETECT 3-DIMENSIONAL DISTRIBUTION OF CURRENT DENSITIES FROM MAGNETIC FIELD GENERATED FROM HEART WITH SQUID FLUXMETER, AND SET, AS Ft(x, y, z), 3-DIMENSIONAL CURRENT DENSITY AT TIME t — S1

OBTAIN INTEGRAL VALUE OF P-WAVE ATRIUM PORTION AT TIME t1 TO t2 — S2

$$S(x,y,z) = \sum_{t1}^{t2} Ft(x,y,z)$$

WHERE Smax DENOTES MAXIMUM OF S(x, y, z).

ITERATIVELY EXECUTE NEXT PROCESSING WITH RESPECT TO ALL COMBINATIONS OF 3-DIMENSIONAL COORDINATES x0 TO xmax, y0 TO ymax, AND z0 TO zmax. — S3

PROCESSING FOR DRAWING CUBE OF ATRIUM — S4

x, y, z — S5

A

# FIG.8

A

OBTAIN INTEGRAL VALUE OF QRS-WAVE VENTRICLE
PORTION AT TIME t3 TO t4

$$S(x,y,z) = \sum_{t3}^{t4} Ft(x,y,z)$$

WHERE Smax DENOTES MAXIMUM OF S(x, y, z)

— S6

ITERATIVELY EXECUTE NEXT
PROCESSING WITH RESPECT TO ALL
COMBINATIONS OF 3-DIMENSIONAL
COORDINATES x0 TO xmax, y0 TO ymax, AND z0 TO zmax.

— S7

PROCESSING FOR DRAWING CUBE OF VENTRICLE

— S8

x, y, z

— S9

PERFORM PERSPECTIVE
PROJECTION

— S10

FIG.9

```
                    ┌─────────────────────┐
                    │  PROCESSING FOR      │──── S4
                    │  DRAWING CUBE        │
                    └─────────────────────┘
                              │
                                              ┌── S41
        ┌──────────────────────────────────────────┐
        │ COUNT NUMBER OF VERTEXES HAVING            │
        │ CURRENT-DENSITY HIGHER THAN                │
        │ THRESHOLD AMONG 8 VERTEXES OF              │
        │ SPECIFIC CUBE                              │
        └──────────────────────────────────────────┘
                              │
                                  ┌── S42
                              ╱───────╲          YES
                            ╱   ≦2?    ╲──────────────────────────────────────┐
                              ╲───────╱                                        │
                                  │                                            │
                                 NO                          ┌── S44           │
                                      ┌── S43                                  │
                              ╱───────╲          YES   ┌──────────────────┐    │
                            ╱   3?     ╲──────────────▶│ DRAW POLYGON     │────┤
                              ╲───────╱                │ HAVING TRIANGLE  │    │
                                  │                    └──────────────────┘    │
                                 NO                          ┌── S46           │
                                      ┌── S45                                  │
                              ╱───────╲          YES   ┌──────────────────┐    │
                            ╱   4?     ╲──────────────▶│ DRAW POLYGON     │────┤
                              ╲───────╱                │ HAVING TRIANGLE  │    │
                                  │                    │ OR TETRAGON      │    │
                                 NO                    └──────────────────┘    │
                                      ┌── S47                ┌── S48           │
                              ╱───────╲          YES   ┌──────────────────┐    │
                            ╱   5?     ╲──────────────▶│ DRAW POLYGON     │────┤
                              ╲───────╱                │ HAVING TRIANGLE  │    │
                                  │                    └──────────────────┘    │
                                 NO                          ┌── S50           │
                                      ┌── S49                                  │
                              ╱───────╲          YES   ┌──────────────────┐    │
                            ╱   6?     ╲──────────────▶│ DRAW POLYGON     │────┤
                              ╲───────╱                │ HAVING TRIANGLE  │    │
                                  │                    │ OR TETRAGON      │    │
                                 NO                    └──────────────────┘    │
                                      ┌── S51                ┌── S52           │
                              ╱───────╲          YES   ┌──────────────────┐    │
                            ╱   7?     ╲──────────────▶│ DRAWN POLYGON    │────┤
                              ╲───────╱                │ HAVING TRIANGLE  │    │
                                  │                    └──────────────────┘    │
                                 NO                                            │
                                  │◀──────────────────────────────────────────┘
                                  │
                          ┌───────────────┐
                          │      END      │
                          └───────────────┘
```

## FIG.10

## FIG.11A

## FIG.11B

## FIG.11C

FIG.12A

FIG.12B

FIG.13A

FIG.13B

FIG.14

## FIG.15

## FIG.16

FIG.17

FIG.18

FIG.19

INTEGRAL RANGE OF ATRIUM
INTEGRAL RANGE OF VENTRICLE

FIG.20

## FIG.21

METHOD FOR DISPLAYING 3-DIMENSIONAL DISTRIBUTION OF QRS-DIFFERENCE

DETECT MAGNETIC FIELD WAVEFORM FROM MAGNETIC FIELD GENERATED FROM HEART WITH SQUID FLUXMETER —S11

OBTAIN ADDICTIVELY-AVERAGED WAVEFORM OF MAGNETOCARDIOGRAPHY CORRESPONDING TO 64-ch WITH R-TRIGGERS IN ELECTROCARDIOGRAM, AND DETECT 3-DIMENSIONAL DISTRIBUTION OF CURRENT DENSITIES OF RESULTANT DATA WITH SPATIAL FILTER. DEFINE, AS Ft(x, y, z), 3-DIMENSIONAL CURRENT DENSITY AT TIME t. —S12

DETECT 3-DIMENSIONAL DISTRIBUTION OF CURRENT DENSITIES BY USING SPATIAL FILTER EVEN TO ADDITIVELY-AVERAGED WAVEFORM OF MAGNETOCARDIOGRAPHY CORRESPONDING TO 64-ch OF TARGET GROUP.
AT THIS TIME, DEFINE AND STORE, AS Ct(x, y, z), AVERAGE OF 3-DIMENSIONAL CURRENT DENSITIES OF TARGET GROUP AT TIME t TO STORAGE UNIT.

ITERATIVELY EXECUTE NEXT PROCESSING WITH RESPECT TO ALL COMBINATIONS OF 3-DIMENSIONAL COORDINATES x0 TO xmax, y0 TO ymax, AND z0 TO zmax. —S13

OBTAIN INTEGRAL VALUE OF Ft(x, y, z) AND Ct(x, y, z) FOR INTERVALS TO BE COMPARED (QRS INTERVAL AS INITIAL ONE), AND DEFINE VALUES AS S(x, y, z) AND SC(x, y, z), RESPECTIVELY. —S14

x、y、z —S15

DEFINE MAXIMUM OF S(x, y, z) AT EACH POINT AS Smax. DEFINE MAXIMUM SC(x, y, z) AT EACH POINT AS SCmax. —S16

( A )

## FIG.22

A

PERFORM SUBTRACTION BETWEEN S AND SC AT ALL POINTS AND DEFINE RESULT AS D(x, y, z).
$D(x, y, z) = SC(x-cx, y-cy, z-cz) \times Smax/SCmax - S(x, y, z)$
WHERE cx, cy, and cz ARE ARBITRARY VALUES FOR CORRECTING SPATIAL INFORMATION.  — S17

DEFINE MAXIMUM OF D(x, y, z) AT EACH POINT AS Dmax  — S18

ITERATIVELY EXECUTE NEXT PROCESSING WITH RESPECT TO ALL COMBINATIONS OF 3-DIMENSIONAL COORDINATES x0 TO xmax, y0 TO ymax, AND z0 TO zmax.  — S19

PROCESSING FOR DRAWING QRS-DIFFERENCE  — S20

x、y、z  — S21

PERFORM PERSPECTIVE PROJECTION  — S22

FIG.23A

FIG.23B

## FIG.24A

## FIG.24B

EP 1 769 741 B1

FIG.25A

FIG.25B

48

FIG.26A

FIG.26B

# FIG.27

METHOD FOR DISPLAYING 3-DIMENSIONAL DISTRIBUTION WITH T-CAD

DETECT MAGNETIC-FIELD WAVEFORM FROM MAGNETIC FIELD GENERATED FROM HEART WITH SQUID FLUXMETER. — S61

OBTAIN ADDITIVELY-AVERAGED WAVEFORMS OF MAGNETOCARDIOGRAPHY OF 64-ch WITH R-WAVE TRIGGERS.
ADD ABSOLUTES OF LATTER HALF OF ADDITIVELY-AVERAGED WAVEFORMS OF MAGNETOCARDIOGRAPHY OF 64-ch, AND SET TIME HAVING THE LARGEST VALUE AS Tpeak. — S62

DETECT 3-DIMENSIONAL DISTRIBUTION OF CURRENT DENSITIES BY USING SPATIAL FILTER TO ADDITIVELY-AVERAGED WAVEFORM OF MAGNETOCARDIOGRAPHY OF 64-ch.
DEFINE 3-DIMENSIONAL DISTRIBUTION OF CURRENT DENSITIES AT TIME t AS Ft(x, y, z) IN THIS CASE, AND DEFINE x COMPONENT AND y COMPONENT TO FXt(x, y, z) AND FYt(x, y, z), RESPECTIVELY.
SQUARE OF Ft(x, y, z) = SQUARE OF FXt(x, y, z) + SQUARE OF FYt(x, y, z) — S63

ITERATIVELY EXECUTE NEXT PROCESSING WITH RESPECT TO ALL COMBINATIONS OF 3-DIMENSIONAL COORDINATES x0 TO xmax, y0 TO ymax, AND z0 TO zmax — S64

OBTAIN INTEGRAL VALUE FROM Tpeak−50 ms (INITIAL VALUE) TO Tpeak+50 ms (INITIAL VALUE). — S65

$$S(x,y,z) = \sum_{Tpeak-50}^{Tpeak+50} Ft(x,y,z)$$

$$SX(x,y,z) = \sum_{Tpeak-50}^{Tpeak+50} FXt(x,y,z)$$

$$SY(x,y,z) = \sum_{Tpeak-50}^{Tpeak+50} FYt(x,y,z)$$

( A )

## FIG.28

(A)

x、y、z ⌐S66

DEFINE MAXIMUM OF S(x, y, z) AT EACH POINT AS Smax ⌐S67

ITERATIVELY EXECUTE NEXT
PROCESSING WITH RESPECT TO ALL
COMBINATIONS OF 3-DIMENSIONAL ⌐S68
COORDINATES x0 TO xmax, y0 TO ymax, AND z0 TO zmax

DRAWING PROCESSING WITH T-CAD ⌐S69

x、y、z ⌐S70

DRAWING PROCESSING WITH HISTOGRAM ⌐S71

PERFORM PERSPECTIVE PROJECTION ⌐S72

FIG.29

FIG.30A

FIG.30B

FIG.31

## FIG.32A

## FIG.32B

FIG.33A

FIG.33B

FIG.34

CIRCULAR GRAPH    270 DEGREE

RED

180 DEGREE                    0 DEGREE

GREEN

90 DEGREE    BLUE

## FIG.35

METHOD FOR DISPLAY 3-DIMENSIONAL DISTRIBUTION OF RT-DISPERSION

DETECT MAGNETIC-FIELD WAVEFORM FROM MAGNETIC FIELD GENERATED FROM HEART WITH SQUID FLUXMETER. — S81

OBTAIN ADDITIVELY-AVERAGED WAVEFORM MAGNETOCARDIOGRAPHY OF 64-ch WITH R-TRIGGERS OF ELECTROCARDIOGRAM.
OBTAIN AVERAGE BETWEEN RR INTERVAL WITH R-TRIGGERS AND SET AVERAGE AS RR TIME. ADD ABSOLUTES OF T-WAVES OF ADDITIVELY-AVERAGED WAVEFORMS OF MAGNETOCARDIOGRAPHY OF 64-ch, AND DEFINE RESULT HAVING LONGEST TIME AS Tpeak TIME — S82

DETECT 3-DIMENSIONAL DISTRIBUTION OF CURRENT DENSITIES AT TIME t BY USING SPATIAL FILTER TO ADDITIVELY-AVERAGED WAVEFORM OF MAGNETOCARDIOGRAPHY OF 64-ch.
DEFINE 3-DIMENSIONAL DISTRIBUTION OF CURRENT DENSITIES AT TIME t AS Ft(x, y, z) — S83

ITERATIVELY EXECUTE NEXT PROCESSING WITH RESPECT TO ALL COMBINATIONS OF 3-DIMENSIONAL COORDINATES x0 TO xmax, y0 TO ymax, AND z0 TO zmax — S84

YES

WHEN x, y, AND z DO NOT EXIST WITHIN CONTOUR OF VENTRICLE — S85

NO

OBTAIN VALUE OF dv/dt WHEN INCLINATION OF T-WAVES BECOMES MAXIMUM IN PERIOD FROM R TIME +70 ms (INITIAL VALUE) TO Tpeak FROM Ft(x, y, z), CALCULATE P(x, y, z) AS RT TIME, AMD SET, AS Color(x, y, z), DIFFERENCE TIME BETWEEN MAX AND MIN. — S86

x, y, z — S87

A

FIG.36

(A)

DEFINE, AS Pmax, MAX OF P(x, y, z) AT EACH POINT — S88

ITERATIVELY EXECUTE NEXT
PROCESSING WITH RESPECT TO ALL
COMBINATIONS OF 3-DIMENSIONAL — S89
COORDINATES x0 TO xmax, y0 TO ymax, AND z0 TO zmax

PROCESSING FOR DRAWING RT-DISPERSION — S90

x、y、z — S91

PERFORM PERSPECTIVE PROJECTION — S92

FIG.37A

FIG.37B

FIG.38A

FIG.38B

## FIG.39A

## FIG.39B

# FIG.40

MAGNETIC FIELD DISTRIBUTION DATA COMPUTING SECTION (14)

ARITHMETIC OPERATION UNIT (7)

STORAGE UNIT (22)

ANATOMICAL IMAGE DATA GENERATOR (3)

FROM IMAGE DIAGNOSTIC APPARATUS

ELECTRONICARDIOGRAPH (21)

EP 1 769 741 B1

## FIG.41

DISPLAY EXCITATION-PROPAGATING LOCUS BASED ON CARDIAC MAGNETIC FIELD

DETECT 3-DIMENSIONAL DISTRIBUTION OF CURRENT DENSITIES FROM MAGNETIC FIELD GENERATED FROM HEART WITH SQUID FLUXMETER, AND SET, AS Ft(x, y, z), 3-DIMENSIONAL CURRENT DENSITY AT TIME t — S111

ITERATE DURING TIME t=t1 TO t2 OF P-WAVE ATRIUM PORTION. — S112

OBTAIN HIGHEST POINT OF 3-DIMENSIONAL CURRENT DENSITY Ft(x, y, z) AT t, t+1, AND t+2, AND CONNECT 3 POINTS WITH B-SPLINE CURVE. — S113

t — S114

ITERATE DURING TIME t=t3 TO t4 OF QRS-WAVE VENTRICLE PORTION. — S115

OBTAIN HIGHEST POINT OF 3-DIMENSIONAL CURRENT DENSITY Ft(x, y, z) AT t, t+1, AND t+2, AND CONNECT 3 POINTS WITH B-SPLINE CURVE. — S116

t — S117

COMBINE TO CARDIAC CONTOUR — S118

FIG.42A

FIG.42B

FIG.43

FIG.44

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

## Patent documents cited in the description

- JP 2002028143 A
- JP 2002028144 A
- JP 2002028145 A
- EP 1302160 A
- JP 11128224 A
- WO 9815226 A

## Non-patent literature cited in the description

- **KENJI NAKAI et al.** Specification of Infarcted and Ischemic Myocardium by Synthetic Aperture Magnetometry on Magnetocardiography. *Japanese Journal of Electro cardiology,* 2003, vol. 23 (1), 35-44
- **KENJI NAKAI et al.** Visualization of Origin of Source by Spatial Filter Method on Magnetocardiography. *Japanese Journal of Electrocardiology,* 2004, vol. 24 (1), 59-66
- **MASATO YOSHIZAWA et al.** Current Density Imaging of MCG signal by Modified SAM. *Collected Papers of Conference of Japan Biomagnetism and Bioelectromagnetics Society,* 2002, vol. 15, 109
- **M. YOSHIZAWA et al.** Current density imaging of simulated MCG signal by Modified Synthetic Aperture Magnetometry. *BIOMAG 2002,* August 2002
- **KENJI NAKAI et al.** Clinical Application and Utility of Magnetocardiography. *Heart,* 15 July 2004, vol. 36 (7), 549-555
- **KENJI NAKAI et al.** Specification of Infarcted and Ischemic Myocardium by Synthetic Aperture Magnetometry on Magnetocardiography. *Japanese Journal of Electrocardiology,* 2003, vol. 23 (1), 35-44
- Functional Neuroimaging by Synthetic Aperture Magnetometry (SAM)'' in ''Recent Advances in Biomagnetism. **ROBINSON SE ; VRBA J.** Proceedings of the 11th International Conference on Biomagnetism. Tohoku University Press, 302-305
- **KOH HARA ; SHINYA KURIKI.** Science of Cerebric Magetic field-SQIUD Measurement and Medical Applications. Ohmsha, Ltd, 25 January 1997, 117-119